# EUROPEAN PATENT SPECIFICATION

(11) **EP 1 917 970 B1**
(45) Date of publication and mention of the grant of the patent: **15.06.2011**
(21) Application number: 07117445.2
(22) Date of filing: 28.06.2000
(51) Int. Cl.: A61K 38/04, A61K 38/08, A61K 38/10, A01N 63/00

(54) **Hla binding peptides and their uses**
Hla-bindende Peptide und ihre Verwendungen
Peptides se fixant au Hla et leurs utilisations

(30) Priority: 29.06.1999 US 141422 P
(43) Date of publication of application: 07.05.2008
(62) Divisional of application: 00944976.0
(73) Proprietor: Epimmune Inc., San Diego, CA 92121 (US)
(72) Inventor: Sette, Alessandro, La Jolla, CA 92037 (US); Sidney, John, San Diego, CA 92130 (US); Southwood, Scott, Santee, CA 92071 (US)
(74) Representative: Inspicos A/S

(56) References cited:
- WO-A-01/62776
- WO-A-94/20127
- WO-A-95/03777
- GUERCIO DEL M-F ET AL: "BINDING OF A PEPTIDE ANTIGEN TO MULTIPLE HLA ALLELES ALLOWS DEFINITION OF AN A2-LIKE SUPERTYPE" JOURNAL OF IMMUNOLOGY, THE WILLIAMS AND WILKINS CO. BALTIMORE, US, vol. 154, 1995, pages 685-693, XP002055570 ISSN: 0022-1767
- GIANFRANI C ET AL: "Human memory CTL response specific for influenza A virus is broad and multispecific." HUMAN IMMUNOLOGY MAY 2000, vol. 61, no. 5, May 2000 (2000-05), pages 438-452, XP002492007 ISSN: 0198-8859
- OGG G S ET AL: "Quantitation of HIV-1-specific cytotoxic T lymphocytes and plasma load of viral RNA." SCIENCE (NEW YORK, N.Y.) 27 MAR 1998, vol. 279, no. 5359, 27 March 1998 (1998-03-27), pages 2103-2106, XP002492008 ISSN: 0036-8075

## Description

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHC molecules are classified as either class I or class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

Investigations of the crystal structure of the human MHC class I molecule, HLA-A2.1, indicate that a peptide binding groove is created by the folding of the α1 and α2 domains of the class I heavy chain (Bjorkman et al., Nature 329:506 (1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC class I has been presented by Rötzschke and Falk (Rötzschke & Falk, Immunol. Today 12:447 (1991).

Sette et al., Proc. Natl. Acad. Sci. USA 86:3296 (1989) showed that MHC allele specific motifs could be used to predict MHC binding capacity. Schaeffer et al., Proc. Natl. Acad. Sci. USA 86:4649 (1989) showed that MHC binding was related to immunogenicity. Several authors (De Bruijn et al., Eur. J Immunol., 21:2963-2970 (1991); Pamer et al., Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population.

Despite the developments in the art, the prior art has yet to provide a useful human epitope-based vaccine or therapeutic agent based on this work. The present invention provides these and other advantages.

WO 94/20127 discloses HLA-A2.1 binding peptides that stimulate CTL responses to HBV, in particular the peptide FLPSDYFPSV. This reference also discloses therapeutic and diagnostic uses of the peptide.

WO 95/03777 discloses HLA binding peptides that induce CTL responses to HBV.

del Guercio M-F et al., J. Immunol. (1995), 154: 685-693, disclose HLA-A2.1 binding peptides that stimulate CTL responses to HBV, in particular the peptide FLPSDFFPSV.

### SUMMARY OF THE INVENTION

The present invention provides compositions comprising at least one peptide, the peptide comprising an epitope consisting of the sequence FQPSDYFPSU.

The present disclosure defines positions within a motif enabling the selection of peptides which will bind efficiently to HLA A2.1. The HLA-A2.1 motif-bearing peptides comprise epitopes of 8-11 amino acids which typically have a first conserved residue at the second position from the N-terminus selected from the group consisting of L, M, I, V, A, T, and Q and a second conserved residue at the C-terminal position selected from the group consisting of V, L, I, A, M, and T.

The primary anchor residues of the HLA-A2.1 motif define the HLA-A2 supermotif; which presence in peptide ligands corresponds to the ability to bind several different HLA-A2 superytpe molecules. The HLA-A2 supermotif comprises peptide ligands with L, I, V, M, A, T, or Q as a primary anchor residue at position 2 and L, I, V, M, A, or T as a primary anchor residue at the C-terminal position of the epitope.

The corresponding family of HLA molecules (*i.e.*, the HLA-A2 supertype that binds these peptides) is comprised of at least: A*0201, A*0202, A*0203, A*0204, A*0205, A*0206, A*0207, A*0209, A*0214, A*6802, and A*6901.

The present disclosure also provides compositions comprising immunogenic peptides having binding motifs for additional MHC Class I molecules. The immunogenic peptides are typically between about 8 and about 11 residues, often 9 or IO residues in length, and comprise conserved residues involved in binding proteins encoded by the appropriate MHC allele. A number of allele specific motifs have been identified.

The HLA-A1 motif is characterized by the presence in peptide ligands of T, S, or M as a primary anchor residue at position 2 and the presence of Y as a primary anchor residue at the C-terminal position of the epitope. An alternative allele-specific A1 motif is characterized by a primary anchor residue at position 3 rather than position 2. This motif is characterized by the presence ofD, E, A, or S as a primary anchor residue in position 3, and a Y as a primary anchor residue at the C-terminal position of the epitope (*see, e.g.,* DiBrino et al., J. Immunol., 152:620, 1994; Kondo et al., Immunogenetics 45:249, 1997; and Kubo et al., I Immunol. I52:3913, 1994 for reviews of relevant data).

The HLA-A3 motif is characterized by the presence in peptide ligands of L, M, V, I, S, A, T, F, C, G, or D as a primary anchor residue at position 2, and the presence of K, Y, R, H, F, or A as a primary anchor residue at the C-terminal position of the epitope (*see, e.g.,* DiBrino et al., Proc. Natl. Acad. Sci USA 90:1508, 1993; and Kubo et al., J. Immunol. 152:3913-3924,1994).

The HLA-A11 motif is characterized by the presence in peptide ligands of V, T, M, L, I, S, A, G, N, C, D, or F as a primary anchor residue in position 2, and K, R, Y, or H as a primary anchor residue at the C-terminal position of the epitope (*see, e.g.,* Zhang et al., Proc. Natl. Acad. Sci USA 90:2217-2221, 1993; and Kubo et al., J. Immunol. 152:3913-3924, 1994).

HLA-A3 and HLA-A11 are members of the HLA-A3 supertype family. The HLA-A3 supermotif is characterized by the presence in peptide ligands of A, L, I, V, M, S, or, T as a primary anchor at position 2, and a positively charged residue, R or K, at the C-terminal position of the epitope, *e.g.*, in position 9 of 9-mers (*see, e.g.,* Sidney et al., Hum. Immunol. 45:79, 1996). Exemplary members of the corresponding family of HLA molecules (the HLA-A3 supertype) that bind the A3 supermotif include A*0301, A*1101, A*3101, A*3301, and A*6801.

The HLA-A24 motif is characterized by the presence in peptide ligands of Y, F, W, or M as a primary anchor residue in position 2, and F, L, I, or W as a primary anchor residue at the C-terminal position of the epitope (*see, e.g.,* Kondo et al., J. Immunol. 155:4307-4312, 1995; and Kubo et al., J. Immunol. 152:3913-3924, 1994).

The disclosure also comprises peptides comprising epitopes containing an HLA-B7 supermotif. The epitopes are 8-11 amino acids in length, often 9 or 10 amino acids in length, and comprise conserved residues of a proline at position 2 and an aromatic residue (*e*.*g*., Y, W, F) or hydrophobic residue (*e.g.*, L, I, V, M, A) at the C-teriminal position of the epitope. Peptides bearing an HLA-B7 supermotif bind to more than one HLA-B7 supertype family member. The corresponding family of HLA molecules that bind the B7 supermotif (i.e., the HLA-B7 supertype) is comprised of at least twenty six HLA-B proteins comprising at least: B*0702, B*0703, B*0704, B*0705, B*1508, B*3501, B*3502, B*3503, B*3504, B*3505, B*3506, B*3507, B*3508, B*5101, B*5102, B*5103, B*5104, B*5105, B*5301, B*5401, B*5501, B*5502, B*5601, B*5602, B*6701, and B*7801 (*see, e.g.,* Sidney, et al., J. Immunol. 154:247, 1995; Barber, et al., Curr. Biol. 5:179, 1995; Hill, et al., Nature 360:434, 1992; Rammensee, et al., Immunogenetics 41:178, 1995).

Epitopes on a number of immunogenic target proteins, *i.e.,* target antigens, have been identified. Examples of suitable antigens include tumor-associated antigens such as tyrosinase related proteins 1 and 2 (TRP1 and TRP), which are frequently associated with melanoma; p53, CEA, Her2/neu, and MAGE, including MAGE1, MAGE2, and MAGE3, which are expressed on a broad range of tumors; prostate cancer-associated antigens such as prostate specific antigen (PSA), human kallikrein (huK2), prostate specific membrane antigen (PSM), and prostatic acid phosphatase (PAP); antigens from viruses such as hepatitis B (*e.g.*, HBV core and surface antigens (HBVc, HBVs)) hepatitis C, Epstein-Barr virus, human immunodeficiency type-1 virus (HIV 1), Kaposi's sarcoma herpes (KSHV), human papilloma virus (HPV), influenza virus, and Lassa virus antigens, *Mycobacterium tuberculosis* (MT) antigens, trypanosome, *e.g., Trypansoma cruzi* (T. cruzi), antigens such as surface antigen (TSA), and malaria antigens.

### DEFINITIONS

The term "peptide" is used interchangeably with "oligopeptide" in the present specification to designate a series of residues, typically L-amino acids, connected one to the other, typically by peptide bonds between the α-amino and carboxyl groups of adjacent amino acids. The preferred CTL-inducing peptides of the invention are 13 residues or less in length and usually consist of between about 8 and about 11 residues, preferably 9 or 10 residues.

With regard to a particular amino acid sequence, an "epitope" is a set of amino acid residues which is involved in recognition by a particular immunoglobulin, or in the context of T cells, those residues necessary for recognition by T cell receptor proteins and/or Major Histocompatibility Complex (MHC) receptors. In an immune system setting, *in vivo* or *in vitro,* an epitope is the collective features of a molecule, such as primary, secondary and tertiary peptide structure, and charge, that together form a site recognized by an immunoglobulin, T cell receptor or HLA molecule. Throughout this disclosure epitope and peptide are often used interchangeably.

It is to be appreciated that protein or peptide molecules that comprise an epitope of the invention as well as additional amino acid(s) are still within the bounds of the invention. In certain embodiments, there is a limitation on the length of a peptide of the disclosure. The embodiment that is length-limited occurs when the protein/peptide comprising an epitope of the invention comprises a region (i.e., a contiguous series of amino acids) having 100% identity with a native sequence. In order to avoid the definition of epitope from reading, *e.g.*, on whole natural molecules, there is a limitation on the length of any region that has 100% identity with a native peptide sequence. Thus, for a peptide comprising an epitope of the disclosure and a region with 100% identity with a native peptide sequence, the region with 100% identity to a native sequence generally has a length of: less than or equal to 600 amino acids, often less than or equal to 500 amino acids, often less than or equal to 400 amino acids, often less than or equal to 250 amino acids, often less than or equal to 100 amino acids, often less than or equal to 85 amino acids, often less than or equal to 75 amino acids, often less than or equal to 65 amino acids, and often less than or equal to 50 amino acids. In certain embodiments, an "epitope" of the disclosure is comprised by a peptide having a region with less than 51 amino acids that has 100% identity to a native peptide sequence, in any increment down to 5 amino acids.

Accordingly, peptide or protein sequences longer than 600 amino acids are within the scope of the invention, so long as they do not comprise any contiguous sequence of more than 600 amino acids that have 100% identity with a native peptide sequence. For any peptide that has five contiguous residues or less that correspond to a native sequence, there is no limitation on the maximal length of that peptide in order to fall within the scope of the invention. It is presently preferred that a CTL epitope be less than 600 residues long in any increment down to eight amino acid residues.

An "immunogenic peptide" or "peptide epitope" is a peptide that comprises an allele-specific motif or supermotif such that the peptide will bind an HLA molecule and induce a CTL. Thus, immunogenic peptides of the disclosure are capable of binding to an appropriate HLA molecule and thereafter inducing a cytotoxic T cell response to the antigen from which the immunogenic peptide is derived.

The term "derived" when used to discuss an epitope is a synonym for "prepared." A derived epitope can be isolated from a natural source, or it can be synthesized in accordance with standard protocols in the art. Synthetic epitopes can comprise artificial amino acids "amino acid mimetics," such as D isomers of natural occurring L amino acids or non-natural amino acids such as cyclohexylalanine. A derived/prepared epitope can be an analog of a native epitope.

Immunogenic peptides are conveniently identified using the algorithms of the invention. The algorithms are mathematical procedures that produce a score which enables the selection of immunogenic peptides. Typically one uses the algorithmic score with a "binding threshold" to enable selection of peptides that have a high probability of binding at a certain affinity and will in turn be immunogenic. The algorithm is based upon either the effects on MHC binding of a particular amino acid at a particular position of a peptide or the effects on binding of a particular substitution in a motif containing peptide.

A binding affinity threshold of about 500 nM (preferably 50 nM or less) typically determines the capacity of a peptide epitope to elicit a CTL response. As used herein, "high affinity" with respect to HLA class I molecules is defined as binding with an IC₅₀, or K_{D} value, of 50 nM or less; "intermediate affinity" is binding with an IC₅₀ or K_{D} value of between about 50 and about 500 nM.

"IC₅₀" is the concentration of peptide in a binding assay at which 50% inhibition of binding of a reference peptide is observed. Given the conditions in which the assays are run (*i.e.*, limiting HLA proteins and labeled peptide concentrations), these values approximate K_{D} values. Assays for determining binding are described in detail, *e.g.*, in PCT publications WO 94/20127 and WO 94/03205. It should be noted that IC₅₀ values can change, often dramatically, if the assay conditions are varied, and depending on the particular reagents used (*e.g.*, HLA preparation, *etc.*). For example, excessive concentrations of HLA molecules will increase the apparent measured IC₅₀ of a given ligand.

Alternatively, binding is expressed relative to a reference peptide. Although as a particular assay becomes more, or less, sensitive, the IC₅₀'s of the peptides tested may change somewhat, the binding relative to the reference peptide will not significantly change. For example, in an assay run under conditions such that the IC₅₀ of the reference peptide increases 10-fold, the IC₅₀ values of the test peptides will also shift approximately 10-fold. Therefore, to avoid ambiguities, the assessment of whether a peptide is a good, intermediate, weak, or negative binder is generally based on its IC₅₀, relative to the IC₅₀ of a standard peptide.

Binding may also be determined using other assay systems including those using: live cells *(e.g.,* Ceppellini et al., Nature 339:392 (1989); Christnick et al., Nature 352:67 (1991); Busch et al., Int. Immunol. 2:443 (1990); Hill et al., J. Immunol. 147:189 (1991); del Guercio et al., J. Immunol. 154:685 (1995)), cell free systems using detergent lysates (*e.g.*, Cerundolo et al., J. Immunol. 21:2069 (1991)), immobilized purified MHC (*e.g.,* Hill et al., J. Immunol. 152, 2890 (1994); Marshall et al., J. Immunol. 152:4946 (1994)), ELISA systems (*e.g.,* Reay et al., EMBO J. 11:2829 (1992)), surface plasmon resonance (*e.g.,* Khilko et al., J. Biol. Chem. 268:15425 (1993)); high flux soluble phase assays (Hammer et al., J. Exp. Med. 180:2353 (1994)), and measurement of class I MHC stabilization or assembly *(e.g.,* Ljunggren et al., Nature 346:476 (1990); Schumacher et al., Cell 62:563 (1990); Townsend et al., Cell 62:285 (1990); Parker et al., J. Immunol. 149:1896 (1992)).

A "conserved residue" is an amino acid which occurs in a significantly higher frequency than would be expected by random distribution at a particular position in a peptide. Typically a conserved residue is one where the MHC structure may provide a contact point with the immunogenic peptide. At least one to three or more, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein, "negative binding residues" are amino acids which if present at certain positions (for example, positions 1, 3 and/or 7 of a 9-mer) will result in a peptide being a nonbinder or poor binder and in turn fail to be immunogenic i.e. induce a CTL response.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues and negative residues.

The binding motif for an allele can be defined with increasing degrees of precision. In one case, all of the conserved residues are present in the correct positions in a peptide and there are no negative residues in positions 1,3 and/or 7.

A "supermotif" is a peptide binding specificity shared by HLA molecules encoded by two or more HLA alleles. Preferably, a supermotif-bearing peptide is recognized with high or intermediate affinity (as defined herein) by two or more HLA molecules.

An "HLA supertype or family", as used herein, describes sets of HLA molecules grouped on the basis of shared peptide-binding specificities. HLA class I molecules that share somewhat similar binding affinity for peptides bearing certain amino acid motifs are grouped into HLA supertypes. The terms HLA superfamily, HLA supertype family, HLA family, and HLA xx-like molecules (where xx denotes a particular HLA type), are synonyms.

The phrases "isolated" or "biologically pure" refer to material which is substantially or essentially free from components which normally accompany it as found in its native state. Thus, the peptides of this invention do not contain materials normally associated with their *in situ* environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

"Pharmaceutically acceptable" refers to a generally non-toxic, inert, and/or physiologically compatible composition.

A "pharmaceutical excipient" comprises a material such as an adjuvant, a carrier, pH-adjusting and buffering agents, tonicity adjusting agents, wetting agents, preservatives, and the like.

"Synthetic peptide" refers to a peptide that is not naturally occurring, but is man-made using such methods as chemical synthesis or recombinant DNA technology.

As used herein, a "vaccine" is a composition that contains at least the peptide of the invention, see, also Tables 5-11. There are numerous embodiments of vaccines in accordance with the invention, such as by a cocktail of one or more peptides; one or more peptides of the invention comprised by a polyepitopic peptide; or nucleic acids that encode such peptides or polypeptides, *e.g.*, a minigene that encodes a polyepitopic peptide. The peptides or polypeptides can optionally be modified, such as by lipidation, addition of targeting or other sequences. HLA class 1-binding peptides of the invention can be linked to HLA class 11-binding peptides, to facilitate activation of both cytotoxic T lymphocytes and helper T lymphocytes. Vaccines can comprise peptide pulsed antigen presenting cells, *e.g.*, dendritic cells.

### DESCRIPTION OF THE PREFERRED EMBODIMENTS

The present invention is based on the determination of allele-specific peptide motifs for human class I MHC (sometimes referred to as HLA) allele subtypes. These motifs are then used to define T cell epitopes from any desired antigen, particularly those associated with human viral diseases, cancers or autoiummune diseases, for which the amino acid sequence of the potential antigen or autoantigen targets is known.

Epitopes on a number of immunogenic target proteins can be identified using the peptides disclosed herein. Examples of antigens include tumor-associated antigens such as TRP1, p53, CEA, Her2/neu, and MAGE, including MAGE1, MAGE2, and MAGE3; prostate cancer-associated antigens such as prostate specific antigen (PSA), human kallikrein (huK2), prostate specific membrane antigen (PSM), and prostatic acid phosphatase (PAP); antigens from viruses such as hepatitis B *(e.g.,* HBV core and surface antigens (HBVc, HBVs)) hepatitis C, Epstein-Barr virus, human immunodeficiency type-1 virus (HIV1), Kaposi's sarcoma herpes (KSHV), human papilloma virus (HPV), influenza virus, and Lassa virus antigens, *Mycobacterium tuberculosis* (MT) antigens, trypanosome, *e.g., Trypansoma cruzi* (T. cruzi), antigens such as surface antigen (TSA), and malaria antigens. The peptides are thus useful in pharmaceutical compositions for both *in vivo* and *ex vivo* therapeutic and diagnostic applications.

Peptides comprising the epitopes from these antigens are synthesized and then tested for their ability to bind to the appropriate MHC molecules in assays using, for example, purified class I molecules and radioiodonated peptides and/or cells expressing empty class I molecules by, for instance, immunofluorescent staining and flow microfluorometry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary *in vitro* or *in vivo* CTL responses that can give rise to CTL populations capable of reacting with virally infected target cells or tumor cells as potential therapeutic agents.

The MHC class I antigens are encoded by the HLA-A, B, and C loci. HLA-A and B antigens are expressed at the cell surface at approximately equal densities, whereas the expression of HLA-C is significantly lower (perhaps as much as 10-fald lower). Each of these loci have a number of alleles. The peptide binding motifs disclosed herein are relatively specific for each allelic subtype.

For peptide-based vaccines, the peptides disclosed herein preferably comprise a motif recognized by an MHC I molecule having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypes, specifically HLA-A2.1, A1, A3.2, and A24.1. Similarly, the majority of the Asian population is encompassed with the addition of peptides binding to a fifth allele HLA-A11.2.

**TABLE 1**

| A Allele/Subtype | N(69)* | A(54) | C(502) |
|---|---|---|---|
| A1 | 10.1(7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9(2) | 27.7(15) | 15.3(77) |
| A24.2 | - | - | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 14.5(10) | - | 0.8(4) |
| Aw36 | 5.9(4) | - | - |

| | | | |
|---|---|---|---|
| Table compiled from DuPont, Immunobiology of HLA, Vol. I, Histocompatibility Testing 1987, Springer-Verlag, New York 1989. * N - negroid; A = Asian; C = caucasoid. Numbers in Parenthesis represent the number of individuals included in the analysis. | | | |

The nomenclature used to describe peptide compounds follows the conventional practice wherein the amino group is presented to the left (the N-terminus) and the carboxyl group to the right (the C-terminus) of each amino acid residue. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

### Identification of Peptides

The large degree of HLA polymorphism is an important factor to be taken into account with the epitope-based approach to vaccine development. To address this factor, epitope selection encompassing identification of peptides capable of binding at high or intermediate affinity to multiple HLA molecules is preferably utilized, most preferably these epitopes bind at high or intermediate affinity to two or more allele-specific HLA molecules.

CTL-inducing peptides of interest for vaccine compositions preferably include those that have an IC₅₀ or binding affinity value for a class I HLA molecule(s) of 500 nM or better (*i.e.*, the value is ≤ 500 nM). For example, peptide binding is assessed by testing the capacity of a candidate peptide to bind to a purified HLA molecule *in vitro.* Peptides exhibiting high or intermediate affinity are then considered for further analysis. Selected peptides are generally tested on other members of the supertype family. In preferred embodiments, peptides that exhibit cross-reactive binding are then used in cellular screening analyses or vaccines.

The relationship between binding affinity for HLA class I molecules and immunogenicity of discrete peptide epitopes on bound antigens was determined for the first time in the art by the present inventors. As disclosed in greater detail herein, higher HLA binding affinity is correlated with greater immunogenicity.

Greater immunogenicity can be manifested in several different ways. Immunogenicity corresponds to whether an immune response is elicited at all, and to the vigor of any particular response, as well as to the extent of a population in which a response is elicited. For example, a peptide might elicit an immune response in a diverse array of the population, yet in no instance produce a vigorous response. In accordance with these principles, close to 90% of high binding peptides have been found to elicit a response and thus be "immunogenic," as contrasted with about 50% of the peptides that bind with intermediate affinity. (See, e.g., Schaeffer et al. PNAS (1988)) Moreover, not only did peptides with higher binding affinity have an enhanced probability of generating an immune response, the generated response tended to be more vigorous than the response seen with weaker binding peptides. As a result, less peptide is required to elicit a similar biological effect if a high affinity binding peptide is used rather than a lower affinity one. Thus, in preferred embodiments of the invention, high affinity binding epitopes are used.

The correlation between binding affinity and immunogenicity was analyzed by the present inventors by two different experimental approaches (*see, e.g.,* Sette, et al., J. Immunol. 153:5586-5592 (1994)). In the first approach, the immunogenicity of potential epitopes ranging in HLA binding affinity over a 10,000-fold range was analyzed in HLA-A*0201 transgenic mice. In the second approach, the antigenicity of approximately 100 different hepatitis B virus (HBV)-derived potential epitopes, all carrying A*0201 binding motifs, was assessed by using PBL from acute hepatitis patients. Pursuant to these approaches, it was determined that an affinity threshold value of approximately 500 nM (preferably 50 nM or less) determines the capacity of a peptide epitope to elicit a CTL response. These data are true for class I binding affinity measurements for naturally processed peptides and for synthesized T cell epitopes. These data also indicate the important role of determinant selection in the shaping of T cell responses (*see, e.g.,* Schaeffer et al. Proc. Natl. Acad. Sci. USA 86:4649-4653 (1989)).

Definition of motifs specific for different class I alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs. The epitopic sequences are then synthesized. The capacity to bind MHC Class molecules is measured in a variety of different ways. One means is a Class I molecule binding assay as described in the references, noted above. Other alternatives described in the literature include inhibition of antigen presentation (Sette, et al., J. Immunol. 141:3893 (1991), *in vitro* assembly assays (Townsend, et al., Cell 62:285 (1990), and FACS based assays using mutated ells, such as RMA-S (Melief, et al., Eur. J. Immunol. 21:2963 (1991)).

A number of protocols can be used to isolate HLA molecules for use in in binding assays. For example, allele-specific mAb reagents can be used for the affinity purification of the HLA-A, HLA-B1, and HLA-C molecules. Several mAb reagents for the isolation of HLA-A molecules are available (*see* Table 2). Thus, for each of the targeted HLA-A alleles, reagents are available that may be used for the direct isolation of the HLA-A molecules. Affinity columns prepared with these mAbs using standard techniques are successfully used to purify the respective HLA-A allele products. In addition to allele-specific mAbs, broadly reactive anti-HLA-A, B, C mAbs, such as W6/32 and B9.12.1, and one anti-HLA-B, C mAb, B1.23.2, could be used in alternative affinity purification protocols as described in previous applications.

**TABLE 2**

| ANTIBODY REAGENTS | | |
|---|---|---|
| anti-HLA | Name | |
| HLA-A1 | 12/18 | |
| HLA-A2 | BB7.2 | |
| HLA-A3 | GAPA3 | (ATCC, HB 122) |
| HLA-11, 24.1 | A11.1M | (ATCC, HB164) |
| HLA-A, B, C | W6/32 | (ATCC, HB95) |
| HLA-A, B, C | 89.12.I | (INSERM-CNRS) |
| HLA-B, C | B.1.23.2 | (INSERM-CNRS) |

Next, peptides that test positive in the MHC class I binding assay are assayed for the ability of the peptides to induce specific CTL responses *in vitro.* For instance, Antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp. Med. 166:182 (1987); Boog, Eur. J. Immunol. 18:219 (1988)).

Alternatively, mutant mammalian cell lines that are deficient in their ability to load class I molecules with internally processed peptides, such as the mouse cell lines RMA-S (Kärre, et al.. Nature, 319:675 (1986); Ljunggren, et al., Eur. J Immunol. 21:2963-2970 (1991)), and the human somatic T cell hybrid, T-2 (Cerundolo, et al., Nature 345:449-452 (1990)) and which have been transfected with the appropriate human class I genes are conveniently used, when peptide is added to them, to test for the capacity of the peptide to induce *in vitro* primary CTL responses. Other eukaryotic cell lines which could be used include various insect cell lines such as mosquito larvae (ATCC cell lines CCL 125, 126, 1660,1591, 6585, 6586), silkworm (ATTC CRL 8851), armyworm (ATCC CRL 1711), moth (ATCC CCL 80) and Drosophila cell lines such as a Schneider cell line (*see* Schneider, J. Embryol. Exp. Morphol. 27:353-365 (1927)).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 µM of peptide in serum-free media for 4 hours under appropriate culture conditions. The peptide-loaded antigen-presenting cells are then incubated with the responder cell populations *in vitro* for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

### Preparation of peptides

Peptides that comprise the epitope consisting of the sequence FQPSDYFPSU can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Often, the peptide will be as small as possible while still maintaining substantially all of the biological activity of the large peptide. When possible, it may be desirable to optimize peptides to a length of 8, 9, 10, or 11 amino acid residues, commensurate in size with endogenously processed viral peptides or tumor cell peptides that are bound to MHC class I molecules on the cell surface.

As the coding sequence for peptides of the length contemplated herein can be synthesized by chemical techniques, for example, the phosphotriester method of Matteucci et al., J. Am. Chem. Soc. 103:3185 (1981),

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A Laboratory Manual, Cold Spring Harbor Press, Cold Spring Harbor, New York (1982). For example, a coding sequence encoding a peptide of the invention can be provided with appropriate linkers and ligated into expression vectors commonly available in the art, and the vectors used to transform suitable hosts to produce the desired fusion protein. A number of such vectors and suitable host systems are now available. Expression constructs, i.e., minigenes are described in greater detail in the sections below.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany & Merrifield, The Peptides, Gross & Meienhofer, eds. (N.Y., Academic Press), pp. 1-284 (1979); and Stewart & Young, Solid Phase Peptide Synthesis, (Rockford, III., Pierce), 2d Ed. (1984).

The peptides can also be modified by extending or decreasing the compound's amino acid sequence, e.g., by the addition or deletion of amino acids. The peptides or analogs can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well as many derivatives of L-α-amino acids.

Typically, a series of peptides with single amino acid substitutions are employed to determine the effect of electrostatic charge, hydrophobicity, etc. on binding. For instance, a series of positively charged (*e.g.*, Lys or Arg) or negatively charged *(e.g.,* Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (*e.g.*, hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions are often made in accordance with the following Table 3.

**TABLE 3**

| Original Residue | Exemplary Substitution |
|---|---|
| Ala | Ser |
| Arg | Lys, His |
| Asn | Gln |
| Asp | Glu |
| Cys | Ser |
| Gln | Asn |
| Glu | Asp |
| Gly | Pro |
| His | Lys; Arg |
| Ile | Leu; Val |
| Leu | Ile; Val |
| Lys | Arg; His |
| Met | Leu; Ile |
| Phe | Tyr; Trp |
| Ser | Thr |
| Thr | Ser |
| Trp | Tyr; Phe |
| Tyr | Trp; Phe |
| Val | Ile; Leu |
| Pro | Gly |

Substantial changes in function (*e.g.*, affinity for MHC molecules or T cell receptors) are made by selecting substitutions that are less conservative than those in Table 3, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, *e.g.* leucyl, isoleucyl, phenylalanyl, valyl or alanyl; (b) a residue having an electropositive side chain, *e.g.*, lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, *e.g.*, glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, Chemistry and Biochemistry of Amino Acids, Peptides and Proteins, Vol. VII (Weinstein ed., 1983).

Modifications of peptides with various amino acid mimetics or unnatural amino acids are particularly useful in increasing the stability of the peptide *in vivo.* Stability can be assayed in a number of ways. For instance, peptidases and various biological media, such as human plasma and serum, have been used to test stability. *See, e.g.,* Verhoef et al., Eur. J Drug Metab Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6% aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

Another embodiment for generating effective peptide analogs involves the substitution of residues that have an adverse impact on peptide stability or solubility in, *e.g.*, a liquid environment. This substitution may occur at any position of the peptide epitope. For example, a cysteine (C) can be substituted out in favor of α-amino butyric acid. Due to its chemical nature, cysteine has the propensity to form disulfide bridges and sufficiently alter the peptide structurally so as to reduce binding capacity. Substituting α-amino butyric acid for C not only alleviates this problem, but actually improves binding and crossbinding capability in certain instances *(see, e.g.,* the review by Sette et al., In: Persistent Viral Infections, Eds. R. Ahmed and I. Chen, John Wiley & Sons, England, 1999). Substitution of cysteine with α-amino butyric acid may occur at any residue of a peptide epitope, *i.e.* at either anchor or non-anchor positions.

### Modification of binding activity

The binding activity, particularly modification of binding affinity or cross-reactivity among HLA supertype family members, of peptides can also be altered using analoging. In brief, the analoging strategy utilizes the motifs or supermotifs that correlate with binding to certain HLA molecules. Analog peptides can be created by substituting amino acid residues at primary anchor, secondary anchor, or at primary and secondary anchor positions. Generally, analogs are made for peptides that already bear a motif or supermotif. For a number of the motifs or supermotifs, residues are defined which are deleterious to binding to allele-specific HLA molecules or members ofHLA supertypes that bind the respective motif or supermotif (*see,* e.g., Rupert et al. Cell 74:929, 1993; Sidney, J. et al., Hu. Immunol. 45:79, 1996; and Sidney et al.; Sidney, et al., J. Immunol. 154:247, 1995). Accordingly, removal of such residues that are detrimental to binding can be performed in accordance with the present invention. For example, in the case of the A3 supertype, when all peptides that have such deleterious residues are removed from the population of peptides used in the analysis, the incidence of cross-reactivity increased from 22% to 37% *(see, e.g.,* Sidney, J. et al., Hu. Immunol. 45:79, 1996).

Thus, one strategy to improve the cross-reactivity of peptides within a given supermotif is simply to delete one or more of the deleterious residues present within a peptide and substitute a small "neutral" residue such as Ala (that may not influence T cell recognition of the peptide). An enhanced likelihood of cross-reactivity is expected if, together with elimination of detrimental residues within a peptide, "preferred" residues associated with high affinity binding to an allele-specific HLA molecule or to multiple HLA molecules within a superfamily are inserted.

To ensure that an analog peptide, when used as a vaccine, actually elicits a CTL response to the native epitope *in vivo,* the analog peptide may be used to induce T cells *in vitro* from individuals of the appropriate HLA allele. Thereafter, the immunized cells' capacity to lyse wild type peptide sensitized target cells is evaluated. Alternatively, evaluation of the cells' activity can be evaluated by monitoring IFN release. Each of these cell monitoring strategies evaluate the recognition of the APC by the CTL. It will be desirable to use as antigen presenting cells, typically cells that have been either infected, or transfected with the appropriate genes to establish whether endogenously produced antigen is also recognized by the T cells induced by the analog peptide. It is to be noted that peptide/protein-pulsed dendritic cells can be used to present whole protein antigens for both HLA class I and class II.

Another possibility is to create analogs of weak binding peptides, to thereby ensure adequate numbers of cellular binders. Class I binding peptides exhibiting binding affinities of 500-5000 nM, and carrying an acceptable but suboptimal primary anchor residue at one or both positions can be "fixed" by substituting preferred anchor residues in accordance with the respective supertype. The analog peptides can then be tested for binding and/or cross-binding capacity.

Another possibility is to create analogs of peptides that are already cross-reactive binders and are vaccine candidates, but which bind weakly to one or more alleles of a supertype. If the cross-reactive binder carries a suboptimal residue (less preferred or deleterious) at a primary or secondary anchor position, the peptide can be analoged by substituting out a deleterious residue and replacing it with a preferred or less preferred one, or by substituting out a less preferred reside and replacing it with a preferred one. The analog peptide can then be tested for cross-binding capacity.

### MHC molecule sources

A large number of cells with defined MHC molecules, particularly MHC Class I molecules, are known and readily available for use as a source of MHC molecules, *e.g.*, for binding assays. For example, human EBV-transformed B cell lines have been shown to be excellent sources for the preparative isolation of class I and class II MHC molecules. Well-characterized cell lines are available from private and commercial sources, such as American Type Culture Collection ("Catalogue of Cell Lines and Hybridomas," 6th edition (1988) Rockville, Maryland, U.S.A.); National Institute of General Medical Sciences 1990/1991 Catalog of Cell Lines (NIGMS) Human Genetic Mutant Cell Repository, Camden, NJ; and ASHI Repository, Brigham and Women's Hospital, 75 Francis Street, Boston, MA 02115. Table 4 lists some B cell lines suitable for use as sources for HLA-A alleles. All of these cell lines can be grown in large batches and are therefore useful for large scale production of MHC molecules. One of skill will recognize that these are merely exemplary cell lines and that many other cell sources can be employed. Similar EBV B cell lines homozygous for HLA-B and HLA-C could serve as sources for HLA-B and HLA-C alleles, respectively.

**TABLE 4**

| HUMAN CELL LINES (HLA-A SOURCES) | |
|---|---|
| HLA-A allele | B cell line |
| A1 | MAT |
| | COX (9022) |
| | STEINLIN (9087) |
| A2.1 | JY |
| A3.2 | EHM (9080) |
| | HO301 (9055) |
| | GM3107 |
| A24.1 | KT3(9107),TISI (9042) |
| A11 | BVR (GM6828A) |
| | WT100 (GM8602) |
| | WT52 (GM8603) |

### Combinations of CTL and HTL epitopes

The peptides which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, *e.g.*, Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. Exemplary T helper peptides include tetanus toxoid 830-843, influenza 307-319, malaria circumsporozoite 382-398 and 378-389.

### Combination with agents to prime the immune response

In some embodiments it may be desirable to include in the pharmaceutical compositions of the invention at least one component which assists in priming CTL. Lipids have been identified as agents capable of assisting the priming CTL *in vivo* against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P₃CSS) can be used to prime virus specific CTL when covalently attached to an appropriate peptide. See, Deres et al., Nature 342:561-564 (1989). Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH2 acylation, e.g., by alkanoyl (C1-C20) or thioglycolyl acetylation, terminal-carboxyl amidation, e.g., ammonia, methylamine, etc. In some instances these modifications may provide sites for linking to a support or other molecule.

### Vaccine compositions

The of the present invention pharmaceutical and vaccine compositions are useful for administration to mammals, particularly humans, to treat and/or prevent viral infection and cancer. Examples of diseases which can be treated or prevented using the immunogenic peptides of the invention include prostate cancer, hepatitis B, hepatitis C, HPV infection, AIDS, renal carcinoma, cervical carcinoma, lymphoma, CMV, malaria, and condlyloma acuminatum.

Vaccines that contain an immunogenically effective amount of one or more peptides as described herein are a further embodiment of the invention. Once appropriately immunogenic epitopes have been defined, they can be delivered by various means, herein referred to as "vaccine" compositions. Such vaccine compositions can include, for example, lipopeptides (*e*.*g*.,Vitiello, A. et al., J. Clin. Invest. 95:341, 1995), peptide compositions encapsulated in poly(DL-lactide-co-glycolide) ("PLG") microspheres *(see, e.g.,* Eldridge, et al., Molec. Immunol. 28:287-294, 1991: Alonso et al., Vaccine 12:299-306, 1994; Jones et al., Vaccine 13:675-681, 1995), peptide compositions contained in immune stimulating complexes (ISCOMS) (*see, e.g.,* Takahashi et al., Nature 344:873-875, 1990; Hu et al., Clin Exp Immunol. 113:235-243, 1998), multiple antigen peptide systems (MAPs) (*see e.g.,* Tam, J. P., Proc. Natl. Acad. Sci. U.S.A. 85:5409-5413, 1988; Tam, J.P., J. Immunol. Methods 196:17-32,1996), viral delivery vectors (Perkus, M. E. et al., In: Concepts in vaccine development, Kaufmann, S. H. E., ed., p. 379, 1996; Chakrabarti, S. et al., Nature 320:535, 1986; Hu, S. L. et al., Nature 320:537, 1986; Kieny, M.-P. et al., AIDS Bio/Technology 4:790,1986; Top, F. H. et al., J. Infect. Dis. 124:148, 1971; Chanda, P. K. et al., Virology 175:535, 1990), particles of viral or synthetic origin *(e.g.,* Kofler, N. et al., J. Immunol. Methods. 192:25, 1996; Eldridge, J. H. et al., Sem. Hematol. 30:16, 1993; Falo, L. D., Jr. et al., Nature Med. 7:649, 1995), adjuvants (Warren, H. S., Vogel, F. R., and Chedid, L. A. Annu. Rev. Immunol. 4:369, 1986; Gupta, R. K. et al., Vaccine 11:293, 1993), liposomes (Reddy, R. et al., J. Immunol. 148:1585, 1992; Rock, K. L., Immunol. Today 17:131, 1996), or, naked or particle absorbed cDNA (Ulmer, J. B. et al., Science 259:1745, 1993; Robinson, H. L., Hunt, L. A., and Webster, R. G., Vaccine 11:957,1993; Shiver, J. W. et al., In: Concepts in vaccine development, Kaufinann, S. H. E., ed., p. 423, 1996; Cease, K. B., and Berzofsky, J. A., Annu. Rev. Immunol. 12:923, 1994 and Eldridge, J. H. et al., Sem. Hematol. 30:16, 1993). Toxin-targeted delivery technologies, also known as receptor mediated targeting, such as those of Avant Immunotherapeutics, Inc. (Needham, Massachusetts) may also be used.

Vaccine compositions described herein include nucleic acid-mediated modalities. DNA or RNA encoding one or more of the peptides of the invention can also be administered to a patient. This approach is described, for instance, in Wolff et. al., Science 247:1465 (1990) as well as U.S. Patent Nos. 5,580,859; 5,589,466; 5,804,566; 5,739,118; 5,736,524; 5,679,647; WO 98/04720; and in more detail below. Examples of DNA-based delivery technologies include "naked DNA", facilitated (bupivicaine, polymers, peptide-mediated) delivery, cationic lipid complexes, and particle-mediated ("gene gun") or pressure-mediated delivery *(see, e.g.,* U.S. Patent No. 5,922,687).

For therapeutic or prophylactic immunization purposes, the peptides can be expressed by viral or bacterial vectors. Examples of expression vectors include attenuated viral hosts, such as vaccinia or fowlpox. This approach involves the use of vaccinia virus, for example, as a vector to express nucleotide sequences that encode the peptides of the invention. Upon introduction into an acutely or chronically infected host or into a non-infected host, the recombinant vaccinia virus expresses the immunogenic peptide, and thereby elicits a host CTL and/or HTL response. Vaccinia vectors and methods useful in immunization protocols are described in, *e.g.*, U.S. Patent No. 4,722,848. Another vector is BCG (Bacille Calmette Guerin). BCG vectors are described in Stover et al., Nature 351:456-460 (1991). A wide variety of other vectors useful for therapeutic administration or immunization of the peptides of the invention, *e.g.* adeno and adeno-associated virus vectors, retroviral vectors, *Salmonella typhi* vectors, detoxified anthrax toxin vectors, and the like, will be apparent to those skilled in the art from the description herein.

Furthermore, vaccines in accordance with the invention can encompass one or more of the peptides. Accordingly, a peptide can be present in a vaccine individually. Alternatively, the peptide can be individually linked to its own carrier; alternatively, the peptide can exist as a homopolymer comprising multiple copies of the same peptide, or as a heteropolymer of various peptides. Polymers have the advantage of increased immunological reaction and, where different peptide epitopes are used to make up the polymer, the additional ability to induce antibodies and/or CTLs that react with different antigenic determinants of the pathogenic organism or tumor-related peptide targeted for an immune response. The composition may be a naturally occurring region of an antigen or may be prepared, *e.g.*, recombinantly or by chemical synthesis.

Carriers that can be used with vaccines of the invention are well known in the art, and include, *e.g.*, thyroglobulin, albumins such as human serum albumin, tetanus toxoid, polyamino acids such as poly L-lysine, poly L-glutamic acid, influenza, hepatitis B virus core protein, and the like. The vaccines can contain a physiologically tolerable (*i.e.*, acceptable) diluent such as water, or saline, preferably phosphate buffered saline. The vaccines also typically include an adjuvant. Adjuvants such as incomplete Freund's adjuvant, aluminum phosphate, aluminum hydroxide, or alum are examples of materials well known in the art. Additionally, CTL responses can be primed by conjugating peptides of the invention to lipids, such as tripalmitayl-S-glycerylcysteinlyseryl- serine (P₃CSS).

Upon immunization with a peptide composition in accordance with the invention, via injection, aerosol, oral, transdermal, transmucosal, intrapleural, intrathecal, or other suitable routes, the immune system of the host responds to the vaccine by producing large amounts of CTLs specific for the desired antigen. Consequently, the host becomes at least partially immune to later infection, or at least partially resistant to developing an ongoing chronic infection, or derives at least some therapeutic benefit when the antigen was tumor-associated.

In certain embodiments, components that induce T cell responses are combined with component that induce antibody responses to the target antigen of interest. combine class I peptide vaccines of the invention with vaccines which induce or facilitate neutralizing antibody responses to the target antigen of interest, particularly to viral envelope antigens. A preferred embodiment of such a composition comprises class I and class II epitopes in accordance with the invention. An alternative embodiment of such a composition comprises a class I epitope in accordance with the invention, along with a PADRE™ (Epimmune, San Diego, CA) molecule (described, for example, in U.S. Patent Number 5,736,142).

### Minigenes

A means of administering nucleic acids encoding the peptides disclosed herein uses minigene constructs encoding multiple epitopes of the invention. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, a leader (signal) sequence, and an endoplasmic reticulum retention signal. In addition, MHC presentation of CTL epitopes may be improved by including synthetic (*e.g.* poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. he ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E. coli* origin of replication; and an *E. coli* selectable marker (*e.g.* ampicillin or kanamycin resistance). Numerous promoters can be used for this purpose, *e.g.*, the human cytomegalovirus (hCMV) promoter. See, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

A bicistronic expression vector, to allow production of the minigene-encoded epitopes and a second protein included to enhance or decrease immunogenicity can be used. Examples of proteins or polypeptides that could beneficially enhance the immune response if co-expressed include cytokines (*e.g.*, IL2, IL12, GM-CSF), cytokine-inducing molecules (*e.g.* LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (*e.g.* TGF-β) may be beneficial in certain diseases.

Once an expression vector is selected, the minigene is cloned into the polylinker region downstream of the promoter. This plasmid is transformed into an appropriate E. coli strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in E. coli, followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted above, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 51 Cr release, indicates production of MHC presentation of minigene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

### Ex vivo administration of epitopes

An embodiment of a vaccine composition in accordance with the invention comprises *ex vivo* administration of a cocktail of epitope-bearing peptides to PBMC, or isolated DC therefrom, from the patient's blood. After pulsing the DC with peptides and prior to reinfusion into patients, the DC are washed to remove unbound peptides. In this embodiment, a vaccine comprises peptide-pulsed DCs which present the pulsed peptide epitopes in HLA molecules on their surfaces.

Dendritic cells can also be transfected, *e.g.*, with a minigene comprising nucleic acid sequences encoding the epitopes, in order to elicit immune responses. Vaccine compositions can be created *in vitro,* following dendritic cell mobilization and harvesting, whereby loading of dendritic cells occurs *in vitro.*

Antigenic peptides are used to elicit a CTL response *ex vivo,* as well. The resulting CTL cells, can be used to treat chronic infections, or tumors in patients that do not respond to other conventional forms of therapy, or will not respond to a therapeutic vaccine peptide or nucleic acid in accordance with the invention. *Ex vivo* CTL or HTL responses to a particular antigen (infectious or tumor-associated antigen) are induced by incubating in tissue culture the patient's, or genetically compatible, CTL or HTL precursor cells together with a source of antigen-presenting cells (APC), such as dendritic cells, and the appropriate immunogenic peptide. After an appropriate incubation time (typically about 7-28 days), in which the precursor cells are activated and expanded into effector cells, the cells are infused back into the patient, where they will destroy their specific target cell (an infected cell or a tumor cell). Transfected dendritic cells may also be used as antigen presenting cells.

### Administration of vaccine compositions

For pharmaceutical compositions, the compositions of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose." Amounts effective for this use will depend on, e.g., the peptide composition, the manner of administration, the stage and seventy of the disease being treated, the weight and general state of health of the patient, and the judgment of the prescribing physician, but generally range for the initial immunization (that is for therapeutic or prophylactic administration) from about 1.0 µg to about 50,000 µg of peptide for a 70 kg patient, followed by boosting dosages of from about 1.0 µg to about 10,000 µg of peptide pursuant to a boosting regimen over weeks to months depending upon the patient's response and condition by measuring specific CTL activity in the patient's blood. It must be kept in mind that the compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 µg to about 50,000 µg, preferably about 5 µg to 10,000 µg for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

The peptides may also be administered via liposomes, which target the peptides to a particular cells tissue, such as lymphoid tissue. Liposomes are also useful in increasing the half-life of the peptides. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired peptide can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4,501,728,4,837,028, and 5,019,369.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

In some instances it may be desirable to combine the peptide vaccines of the invention with vaccines which induce neutralizing antibody responses to the virus of interest, particularly to viral envelope antigens.

Antigenic peptides may be used to elicit CTL *ex vivo,* as well. The resulting CTL, can be used to treat chronic infections (viral or bacterial) or tumors in patients that do not respond to other conventional forms of therapy, or will not respond to a peptide vaccine approach of therapy. *Ex vivo* CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell). In order to optimize the *in vitro* conditions for the generation of specific cytotoxic T cells, the culture of stimulator cells is maintained in an appropriate serum-free medium.

Prior to incubation of the stimulator cells with the cells to be activated, e.g., precursor CD8+ cells, an amount of antigenic peptide is added to the stimulator cell culture, of sufficient quantity to become loaded onto the human Class I molecules to be expressed on the surface of the stimulator cells. In the present invention, a sufficient amount of peptide is an amount that will allow about 200, and preferably 200 or more, human Class I MHC molecules loaded with peptide to be expressed on the surface of each stimulator cell. Often, the stimulator cells are incubated with >20 µg/ml peptide.

Resting or precursor CD8+ cells are then incubated in culture with the appropriate stimulator cells for a time period sufficient to activate the CD8+ cells. Preferably, the CD8+ cells are activated in an antigen-specific manner. The ratio of resting or precursor CD8+ (effector) cells to stimulator cells may vary from individual to individual and may further depend upon variables such as the amenability of an individual's lymphocytes to culturing conditions and the nature and severity of the disease condition or other condition for which the within-described treatment modality is used. Preferably, however, the lymphocyte:stimulator cell ratio is in the range of about 30:1 to 300:1. The effector/stimulator culture may be maintained for as long a time as is necessary to stimulate a therapeutically useable or effective number of CD8+ cells.

The induction of CTL *in vitro* requires the specific recognition of peptides that are bound to allele specific MHC class I molecules on APC. The number of specific MHC/peptide complexes per APC is crucial for the stimulation of CTL, particularly in primary immune responses. While small amounts of peptide/MHC complexes per cell are sufficient to render a cell susceptible to lysis by CTL, or to stimulate a secondary CTL response, the successful activation of a CTL precursor (pCTL) during primary response requires a significantly higher number of MHC/peptide complexes. Peptide loading of empty major histocompatability complex molecules on cells allows the induction of primary cytotoxic T lymphocyte responses. Peptide loading of empty major histocompatability complex molecules on cells enables the induction of primary cytotoxic T lymphocyte responses.

Since mutant cell lines do not exist for every human MHC allele, it is advantageous to use a technique to remove endogenous MHC-associated peptides from the surface of APC, followed by loading the resulting empty MHC molecules with the immunogenic peptides of interest. The use of non-transformed (non-tumorigenic), non-infected cells, and preferably, autologous cells of patients as APC is desirable for the design of CTL induction protocols directed towards development of ex vivo CTL therapies. This application discloses methods for stripping the endogenous MHC-associated peptides from the surface af APC followed by the loading of desired peptides.

A stable MHC class I molecule is a trimeric complex formed of the following elements: 1) a peptide usually of 8 - 10 residues, 2) a transmembrane heavy polymorphic protein chain which bears the peptide-binding site, and 3) a non-covalently associated non-polymorphic light chain, β2microglobulin. Removing the bound peptides and/or dissociating the β2microglobulin from the complex renders the MHC class I molecules nonfunctional and unstable, resulting in rapid degradation. All MHC class I molecules isolated from PBMCs have endogenous peptides bound to them. Therefore, the first step is to remove all endogenous peptides bound to MHC class I molecules on the APC without causing their degradation before exogenous peptides can be added to them.

Two possible ways to free up MHC class I molecules of bound peptides include lowering the culture temperature from 37°C to 26°C overnight to destablize β2microglobulin and stripping the endogenous peptides from the cell using a mild acid treatment. The methods release previously bound peptides into the extracellular environment allowing new exogenous peptides to bind to the empty class I molecules. The cold-temperature incubation method enables exogenous peptides to bind efficiently to the MHC complex, but requires an overnight incubation at 26°C which may slow the cell's metabolic rate. It is also likely that cells not actively synthesizing MHC molecules (*e.g.*, resting PBMC) would not produce high amounts of empty surface MHC molecules by the cold temperature procedure.

Harsh acid stripping involves extraction of the peptides with trifluoroacetic acid, pH 2, or acid denaturation of the immunoaffinity purified class I-peptide complexes. These methods are not feasible for CTL induction, since it is important to remove the endogenous peptides while preserving APC viability and an optimal metabolic state which is critical for antigen presentation. Mild acid solutions of pH 3 such as glycine or citrate-phosphate buffers have been used to identify endogenous peptides and to identify tumor associated T cell epitopes. The treatment is especially effective, in that only the MHC class I molecules are destabilized (and associated peptides released), while other surface antigens remain intact, including MHC class II molecules. Most importantly, treatment of cells with the mild acid solutions do not affect the cell's viability or metabolic state. The mild acid treatment is rapid since the stripping of the endogenous peptides occurs in two minutes at 4°C and the APC is ready to perform its function after the appropriate peptides are loaded. The technique is utilized herein to make peptide-specific APCs for the generation of primary antigen-specific CTL. The resulting APC are efficient in inducing peptide-specific CD8+ CTL.

Activated CD8+ cells may be effectively separated from the stimulator cells using one of a variety of known methods. For example, monoclonal antibodies specific for the stimulator cells, for the peptides loaded onto the stimulator cells, or for the CD8+ cells (or a segment thereof) may be utilized to bind their appropriate complementary ligand. Antibody-tagged molecules may then be extracted from the stimulator-effector cell admixture via appropriate means, e.g., via well-known immunoprecipitation or immunoassay methods.

Effective, cytotoxic amounts of the activated CD8+ cells can vary between *in vitro* and *in vivo* uses, as well as with the amount and type of cells that are the ultimate target of these killer cells. The amount will also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5X10⁶-5X10⁷ cells used in mice.

Preferably, as discussed above, the activated CD8+ cells are harvested from the cell culture prior to administration of the CD8+ cells to the individual being treated. It is important to note, however, that unlike other present and proposed treatment modalities, the present method uses a cell culture system that is not tumorigenic. Therefore, if complete separation of stimulator cells and activated CD8+ cells is not achieved, there is no inherent danger known to be associated with the administration of a small number of stimulator cells, whereas administration of mammalian tumor-promoting cells may be extremely hazardous.

Methods of re-introducing cellular components are known in the art and include procedures such as those exemplified in U.S. Patent No. 4,844,893 to Honsik, et al. and U.S. Patent No. 4,690,915 to Rosenberg. For example, administration of activated CD8+ cells via intravenous infusion is appropriate.

### Use of Peptide Epitopes as Diagnostic Agents for Evaluating Immune Responses

HLA class I and class II binding peptides can be used as reagents to evaluate an immune response. The evaluated immune response can be induced by any immunogen. For example, the immunogen may result in the production of antigen-specific CTLs or HTLs that recognize the peptide epitope(s) employed as the reagent. Thus, a peptide disclosed herein may or may not be used as the immunogen. Assay systems that can be used for such analyses include tetramer-based protocols, staining for intracellular lymphokines, interferon release assays, or ELISPOT assays.

For example, following exposure to a putative immunogen, a peptide can be used in a tetramer staining assay to assess peripheral blood mononuclear cells for the presence of any antigen-specific CTLs. The HLA-tetrameric complex is used to directly visualize antigen-specific CTLs and thereby determine the frequency of such antigen-specific CTLs in a sample of peripheral blood mononuclear cells *(see, e*.*g*., Ogg et al., Science 279:2103-2106, 1998; and Altman et al., Science 174:94-96, 1996).

A tetramer reagent comprising a peptide is generated as follows: A peptide that binds to an HLA molecule is refolded in the presence of the corresponding HLA heavy chain and β2-microglobulin to generate a trimolecular complex. The complex is biotinylated at the carboxyl terminal end of the HLA heavy chain, at a site that was previously engineered into the protein. Tetramer formation is then induced by adding streptavidin. When fluorescently labeled streptavidin is used, the tetrameric complex is used to stain antigen-specific cells. The labeled cells are then readily identified, *e.g.*, by flow cytometry. Such procedures are used for diagnostic or prognostic purposes; the cells identified by the procedure can be used for therapeutic purposes.

Peptides disclosed herein are also used as reagents to evaluate immune recall responses. (*see, e.g.,* Bertoni et al., J. Clin. Invest. 100:503-513, 1997 and Penna et al., J. Exp. Med. 174:1565-1570, 1991.) For example, a PBMC sample from an individual expressing a disease-associated antigen (e.g. a tumor-associated antigen such as CEA, p53, MAGE2/3,HER2neu, or an organism associated with neoplasia such as HPV or HSV) can be analyzed for the presence of antigen-specific CTLs or HTLs using specific peptides. A blood sample containing mononuclear cells may be evaluated by cultivating the PBMCs and stimulating the cells with a peptide of the invention. After an appropriate cultivation period, the expanded cell population may be analyzed, for example, for CTL or for HTL activity.

Thus, the peptides can be used to evaluate the efficacy of a vaccine. PBMCs obtained from a patient vaccinated with an immunogen may be analyzed by methods such as those described herein. The patient is HLA typed, and peptide epitopes that are bound by the HLA molecule(s) present in that patient are selected for analysis. The immunogenicity of the vaccine is indicated by the presence of CTLs and/or HTLs directed to epitopes present in the vaccine.

The peptides disclosed herein may also be used to make antibodies, using techniques well known in the art (see, *e.g.* CURRENT PROTOCOLS IN IMMUNOLOGY, Wiley/Greene, NY; and Antibodies A Laboratory Manual Harlow, Harlow and Lane, Cold Spring Harbor Laboratory Press, 1989). Such antibodies are useful as reagents to determine the presence of disease-associated antigens or may be used therapetucially. Antibodies in this category include those that recognize a peptide when bound by an HLA molecule, *i.e.*, antibodies that bind to a peptide-MHC complex.

### EXAMPLES

The following examples are provided by way of illustration only and not by way of limitation. Those of skill in the art will readily recognize a variety of noncritical parameters that could be changed or modified to yield essentially similar results.

The results of peptides not encompassed by the claims are for comparative purposes.

### Example 1

Class I antigen isolation was carried out as described in the references above. Naturally processed peptides were then isolated and sequenced as described there. An allele-specific motif and algorithms were determined and quantitative binding assays were carried out.

Using the motifs identified above for HLA-A2.1 motif, which also corresponds to the HLA-A2 supermotif, amino acid sequences from a number of antigens were analyzed for the presence of these motifs. Tables 5 and 6 provide the results of these searches.

**Table 5**

| Peptide | Sequence | Source | A*0201 | A2 XRN |
|---|---|---|---|---|
| 1302.01 | FQPSDYFPSV | HBV.core.18.Q2Y6 | 0.32 | 4 |
| 1302.02 | YLLTRILTI | HBV.env.183.Y1 | 0.2 | 1 |
| 1302.03 | FLYTRILTI | HBV.env.183.Y3 | 0.26 | 1 |
| 1302.04 | FLLTYILTI | HBV.env.183.Y5 | 1 | 1 |
| 1302.05 | FLLTRILYI | HBV.env.183.Y8 | 0.37 | 1 |
| 1319.13 | SLCPIRGWAI | Flu.NRAM.75 | 0.0091 | 1 |
| 1334.1 | KVFGSLAFV | Her2/neu.369.V2V9 | 0.25 | 4 |
| 1334.11 | KTFGSLAFV | Her2/neu.369.T2V9 | 0.1424 | 4 |
| F158.01 | CVNGVCWTV | HCV.NS3.1078 | 0.0004 | 0 |
| F158.02 | CANGVCWTV | HCV.NS3.1078.A2 | 0.0015 | 2 |
| F158.03 | CVNGVCWAV | HCV.NS3.1078.A8 | 0.0007 | 0 |
| 1349.03 | SQSDISLEL | mp53.7 | 0.026 | |
| 1349.04 | SLSDISLEL | mp53.7.L2 | 0.41 | |
| 1349.05 | SQSDISLEV | mp53.7.V9 | 0.082 | |
| 1349.06 | SLSDISLEV | mp53.7.L2V9 | 0.5 | |
| 1349.08 | GLPAAQDPV | mp53.65.L2 | 0.0072 | |
| 1349.1 | YLGNYGFHL | mp53.100.L2 | 0.24 | |
| 1349.11 | YQGNYGFHV | mp53.100.V9 | 0.0065 | |
| 1349.12 | YLGNYGFHV | mp53.100.L2V9 | 0.3 | |
| 1349.13 | VMCTYSPPL | mp53.119 | 0.032 | |
| 1349.14 | VMCTYSPPV | mp53.119.V9 | 0.038 | |
| 1349.15 | VLCTYSPPV | mp53.119.L2V9 | 0.036 | |
| 1349.16 | KLFCQLAKT | mp53.129 | 0.0073 | |
| 1349.27 | ALPPAGSRV | mp53.146.L2 | 0.029 | |
| 1349.31 | VLPYEPPEV | mp53.214.L2V9 | 0.01 | |
| 1295.06 | LLGRDSFEV | mp53.261 | 0.2 | |
| 1349.33 | ALPWPLSSFV | mp53.85.L2 | 0.029 | |
| 1349.34 | FLQSGTAKSV | mp53.110 | 0.017 | |
| 1349.35 | FLESGTAKSV | mp53.110.E3 | 0.0059 | |
| 1349.36 | SVMCTYSPPL | mp53.118 | 0.0078 | |
| 1349.37 | SLMCTYSPPL | mp53.118.L2 | 0.064 | |
| 1349.38 | SVMCTYSPPV | mp53.118.V10 | 0.014 | |
| 1349.39 | SLMCTYSPPV | mp53.118.L2V10 | 0.11 | |
| 1349.47 | SLSDISLELPL | mp53.7.L2 | 0.029 | |
| 1349.49 | SLSDISLELPV | mp53.7.L2V11 | 0.049 | |
| 1349.55 | KLYQGNYGFHL | mp53.98.L2 | 0.024 | |
| 1349.57 | KLYQGNYGFHV | mp53.98.L2V11 | 0.042 | |
| 1349.59 | KLCPVQLWVSA | mp53.136.L2 | 0.021 | |
| 1349.6 | KTCPVQLWVSV | mp53.136.V11 | 0.0094 | |
| 1349.61 | KLCPVQLWVSV | mp53.136.L2V11 | 0.122 | |
| 1295.01 | GLAPPQHLIRV | mp53.184 | 0.063 | |
| 1323.29 | YMCNSSCMGGM | mp53.233 | 0.0075 | |
| 1349.62 | YMCNSSCMGGV | mp53.233.V11 | 0.14 | |
| 1323.31 | YLCNSSCMGGV | mp53.233.L2V11 | 0.23 | |
| 1349.63 | YLCDSSCMGGV | mp53.233.L2D4V11 | 0.15 | |
| 1317.26 | ITLEDSSGNLL | mp53.252 | 0.0032 | |
| 1349.67 | VLCPELPPGSV | mp53.291.V11 | 0.005 | |
| 1352.01 | IMMGVLVGV | CEA.691.M3 | 0.24 | |
| 1352.02 | IMIGHLVGV | CEA.691.H5 | 0.495 | |
| 1352.03 | KVAEIVHFL | MAGE3.112.15 | 0.049 | |
| 1352.04 | KVAELVWFL | MAGE3.112.W7 | 1.03 | |
| 1368.01 | YVSGANLNV | CEA.605.V2V9 | 0.021 | 4 |
| 1368.02 | KVBPVQLWV | p53.139.V2 | 0.044 | 3 |
| 1368.03 | SVPPPGTRV | p53.149.V2 | 0.0027 | 2 |
| 1368.04 | VVLGVVFGV | Her2/neu.665.V2V9 | 0.29 | 4 |
| 1368.05 | YVQLVFGIEV | MAGE2.157.V2 | 0.017 | 2 |
| 1368.06 | AVVGIMIGV | CEA.687.V2 | 0.17 | 4 |
| 1368.07 | IVIGVLVGV | CEA.691.V2 | 0.0026 | 2 |
| 1368.08 | KVAELVHFV | MAGE3.112.V9 | 0.07 | 4 |

**Table 6**

| AA | Sequence | Source | A*0201 | A*0202 | A*0203 | A*0206 | A*6802 | A2 XRN |
|---|---|---|---|---|---|---|---|---|
| 9 | ALKMTMASV | Flu.NS1.76 | 0.022 | | | | | 1 |
| 9 | ALLKHRFEI | Flu.RRP2.70 | 0.045 | | | | | 1 |
| 9 | CLLQSLQQI | Flu.RRP2.584 | 0.031 | | | | | 1 |
| 9 | FLEESHPGI | Flu.RRP1.94 | 0.044 | | | | | 1 |
| 9 | FLWHV12KRV | Flu.NS1.14 | 0.024 | | | | | 1 |
| 9 | FMYSDFHFI | Flu.RRP2.46 | 1.4 | | | | | 1 |
| 9 | GLISLILQI | Flu.NRAM.18 | 0.13 | | | | | 1 |
| 9 | GMFNMLSTV | Flu.RRP1.410 | 0.18 | | | | | 1 |
| 9 | GMGWLTIGI | Flu.NRAM.172 | 0.038 | | | | | 1 |
| 9 | LLLEVEQEI | Flu.NS2.105 | 0.023 | | | | | 1 |
| 10 | LLMDAMCLSI | Flu.RRP2.283 | 0.13 | | | | | 1 |
| 9 | LMDALKLSI | Flu.RRP2.284 | 0.12 | | | | | 1 |
| 10 | MLLRSAIGQV | Flu.RRP2.548 | 0.014 | | | | | 1 |
| 10 | MLSTVLGVSI | Flu.RRP1.414 | 0.02 | | | | | 1 |
| 10 | NLYNIRNLHI | Flu.RRP1.597 | 0.082 | | | | | 1 |
| 9 | NMLSTVLGV | Flu.RRP1.413 | 0.108 | | | | | 1 |
| 9 | RLIDFLKDV | Flu.RRP1.162 | 0.087 | | | | | 1 |
| 9 | RLNKRSYLI | Flu.RRP1.211 | 0.059 | | | | | 1 |
| 9 | RMQFSSFTV | Flu.RRP3.630 | 0.18 | | | | | 1 |
| 10 | SLCPIRGWAI | Flu.NRAM.75 | 0.026 | | | | | 1 |
| 9 | SLENFRAYV | Flu.RRP2.225 | 0.026 | | | | | 1 |
| 9 | SMIEAESSV | Flu.RRP2.594 | 0.031 | | | | | 1 |
| 9 | WMMAMKYPI | Flu.RRP3.49 | 0.069 | | | | | 1 |
| 10 | FIPSDYFPSV | HBV core 18 analog | 0.18 | 1.5 | 0.41 | 2.8 | 0.06 | 4 |
| 10 | FLKSDYFPSV | HBV core 18 analog | 0.44 | 0.39 | 13 | 0.17 | 0.0022 | 4 |
| 10 | FLPKDYFPSV | HBV core 18 analog | 0.95 | 0.82 | 3.4 | 1.3 | 0.0043 | 4 |
| 10 | FLPSDKFPSV | HBV core 18 analog | 0.58 | 0.7 | 6.8 | 0.39 | 0.016 | 4 |
| 10 | FLPSDYFKSV | HBV core 18 analog | 0.25 | 0.22 | 6.1 | 0.29 | 0.0009 | 4 |
| 10 | FLPSDYFPKV | HBV core 18 analog | 0.14 | 0.18 | 0.21 | 0.25 | 0.0042 | 4 |
| 10 | FLPSDYFPSI | HBV core 18 analog | 0.21 | 0.7 | 0.15 | 0.26 | 0.0039 | 4 |
| 10 | FLPSDYFPSL | HBV core 18 analog | 0.18 | 0.43 | 0.23 | 0.077 | 0.0009 | 4 |
| 10 | FLPSDYFPSP | HBV core 18 analog | 0.023 | 0.0004 | 0.0051 | 0.012 | -0.0001 | 2 |
| 10 | FLPSDYFPST | HBV core 18 analog | 0.033 | 0.045 | 0.027 | 0.1 | -0.0001 | 4 |
| 10 | FLPSDYFPSV | HBV core 18 analog | 1.6 | 2.9 | 1.6 | 2.2 | 0.02 | 4 |
| 10 | FLPSKYFPSV | HBV core 18 analog | 0.6 | 0.75 | 12 | 0.76 | 0.0077 | 4 |
| 10 | FQPSDYFPSV | HBV core 18 analog | 0.26 | 0.11 | 0.49 | 0.41 | 0.0002 | 4 |
| 10 | FTPSI7YFFSV | HBV core 18 analog | 0.013 | 0.78 | 0.025 | 0.31 | 0.11 | 4 |
| 10 | FVGPLLVLQV | HBV.env.171.V2 V10 0 | 0.0056 | 0.089 | 0.058 | 0.18 | 0.035 | 3 |
| 9 | FVLSLGIHV | HBV.pol.562.V2 V9 | 0.12 | 0.011 | 0.0036 | 0.029 | 0.14 | 3 |
| 9 | FVLSLGIHV | HBV.pol.573.V2 V9 | 0.013 | 0.0056 | 0.0006 | 0.0079 | 0.0069 | 2 |
| 10 | FVPSDYFPSV | HBV core 18 analog | 0.065 | 2.2 | 0.1 | 1.7 | 0.1 | 4 |
| 9 | GVLGWSPQV | HBV.env.62.V2V 9 | 0.22 | 0.027 | 0.026 | 0.14 | 0.0009 | 4 |
| 9 | GVLGWSPQV | HBV.env.73.V2V 9 | 0.24 | 0.033 | 0.016 | 0.18 | 0.0008 | 3 |
| 10 | GVSPTVWLSV | HBV.env.359.V2 | 0.025 | 0.056 | 2.1 | 0.07 | 0.015 | 4 |
| 9 | HVYSHPIIV | HBV.pol.1076.V 2V9 | 0.033 | 0.0023 | 0.71 | 0.0031 | 0.071 | 2 |
| 9 | HVYSHPIIV | HBV.pol.502.V2 V9 | 0.021 | 0.0013 | 0.89 | 0.0038 | 0.041 | 2 |
| 10 | KLPSDYFPSV | HBV core 18 analog | 0.43 | 0.75 | 0.72 | 1.7 | 0.0002 | 4 |
| 10 | LVDYQGMLPV | HBV.env.271.V2 | 0.0057 | 0.0044 | 0.02 | 0.02 | 0.0008 | 2 |
| 9 | LVWFHISCV | HBV.core.129.V 2V9 | 0.029 | 0.016 | 0.11 | 0.01 | 0.017 | 4 |
| 9 | MVWYWGPSV | HBV.env.360.V2 V9 | 0.03 | 0.0082 | 0.011 | 0.083 | 0.48 | 2 |
| 9 | PVLPIFFCV | HBV.env.377.V2 V9 | 0.61 | 0.0016 | 0.0007 | 0.13 | 0.0045 | 2 |
| 9 | PVLPIFFCV | HBV.env.388.V2 V9 | 0.35 | 0.0012 | 0.0004 | 0.22 | 0.0054 | 2 |
| 9 | VVQAGFFLV | HBV.env.177.V2 V9 | 0.013 | 0.058 | 0.004 | 0.05 | 0.013 | 3 |
| 9 | VVQAGFFLV | HBV.env.177.V2 V9 | 0.0029 | 0.015 | 0.0045 | 0.13 | 0.011 | 2 |
| 9 | WVLRGTSFV | HBV.pol.770.V2 | 0.017 | 0.0042 | 0.085 | 0.045 | 0.023 | 3 |
| 9 | YVDDVVLGV | HBV.pol.538.V2 V9 | 0.28 | 0.34 | 0.15 | 0.23 | 0.023 | 4 |
| 9 | YVDDVVLGV | HBV.pol.549.V2 V9 | 0.31 | 1.9 | 0.092 | 0.23 | 0.019 | 4 |
| 10 | YVHTLWKAGV | HBV.pol.147.V2 V10 | 0.0028 | 0.016 | 0.16 | 0.0045 | 0.051 | 2 |
| 9 | YVVSFGVWV | HBV.core.147.V 2V9 | 0.092 | 0.056 | 0.31 | 0.058 | 1 | 4 |
| 9 | ALVAYQATV | HCV | 0.078 | 0.007 | 2.4 | 0.025 | 0.0045 | 3 |
| 9 | AVSTGLIHV | HCV.NS1.686.V2 V9 | 0.002 | 0.0052 | 0.03 | 0.036 | 0.0017 | 2 |
| 9 | KLVAYQATV | HCV | 0.12 | 0.016 | 1.8 | 0.054 | 0.0009 | 4 |
| 10 | RVHGLSAFSV | HCV.NS5.2918.V 2V10 | 0.01 | 0.011 | 0.025 | 0.089 | 0.013 | 4 |
| 9 | YIVAYQATV | HCV | 0.15 | 0.076 | 3.7 | 0.15 | 0.26 | 4 |
| 9 | YLKAYQATV | HCV | 0.16 | 0.03 | 3.5 | 0.012 | 0.0036 | 4 |
| 9 | YLQAYQATV | HCV | 0.14 | 0.031 | 2.7 | 0.11 | 0.1 | 4 |
| 9 | YLVAAQATV | HCV | 0.14 | 0.058 | 2.4 | 0.056 | 0.046 | 4 |
| 9 | YLVAKQATV | HCV | 0.05 | 0.0085 | 1.7 | 0.03 | 0.0013 | 3 |
| 9 | YLVAYAATV | HCV | 0.075 | 0.13 | 3.2 | 0.093 | 0.029 | 4 |
| 9 | YLVAYKATV | HCV | 0.16 | 0.011 | 1.5 | 0.019 | 0.0099 | 4 |
| 9 | YLVAYQAFV | HCV | 0.57 | 0.43 | 3.8 | 0.16 | 0.21 | 4 |
| 9 | YLVAYQAICV | HCV | 0.074 | 0.043 | 2.1 | 0.026 | 0.021 | 4 |
| 9 | YLVAYQATF | HCV | 0.022 | 0.0007 | 0.19 | 0.0011 | -0.0002 | 2 |
| 9 | YLVAYQATI | HCV | 0.072 | 0.014 | 2.6 | 0.022 | 0.0063 | 4 |
| 9 | YLVAYQATL | HCV | 0.31 | 0.099 | 3.1 | 0.049 | 0.0052 | 4 |
| 9 | YLVAYQATT | HCV | 0.052 | 0.0012 | 0.49 | 0.0044 | -0.0003 | 2 |
| 9 | YLVAYQKTV | HCV | 0.017 | 0.005 | 1.8 | 0.012 | 0.0071 | 3 |
| 9 | YLVAYQQTV | HCV | 0.13 | 0.079 | 1.6 | 0.068 | 0.011 | 4 |
| 9 | YLVKYQATV | HCV | 0.11 | 0.061 | 2.5 | 0.077 | 0.029 | 4 |
| 9 | YLVQYQATV | HCV | 0.35 | 0.099 | 4.3 | 0.13 | 0.032 | 4 |
| 9 | YQVAYQATV | HCV | 0.28 | 0.18 | 2.7 | 0.11 | 0.0022 | 4 |
| 9 | YTVAYQATV | HCV | 0.18 | 0.077 | 0.61 | 0.14 | 5.8 | 4 |
| 9 | YVVAYQATV | HCV | 0.084 | 0.089 | 1.3 | 0.098 | 4 | 4 |
| 9 | ILKEPVMGV | HIV pol 476 analog | 0.024 | | | | | 1 |
| 9 | ILYEPVHGV | HIV pol 476 analog | 0.2 | | | | | 1 |
| 9 | IMHEPVHGV | HIV pol 476 analog | 0.061 | | | | | 1 |
| 9 | IMKEPVHGV | HIV pol 476 analog | 0.028 | | | | | 1 |
| 9 | IMKEPVYGV | HIV pol 476 analog | 0.027 | | | | | 1 |
| 9 | IMYEPVHGV | HIV pol 476 analog | 0.18 | | | | | 1 |
| 9 | KLTPLCVTV | HIV env 134 analog | 0.32 | | | | | 1 |
| 9 | KMTPLCVTL | HIV env 134 analog | 0.29 | | | | | 1 |
| 9 | KMTPLCVTV | HIV env 134 analog | 0.55 | | | | | 1 |
| 9 | MLKEPVHGV | HIV pol 476 analog | 0.077 | | | | | 1 |
| 9 | MMKEPVHGV | HIV pol 476 analog | 0.069 | | | | | 1 |
| 9 | WLKEPVHGV | HIV pol 476 analog | 0.015 | | | | | 1 |
| 9 | YMKEPVHGV | HIV pol 476 analog | 0.056 | | | | | 1 |
| 9 | LLFGYPVYV | HTLV tax 11 | 0.47 | | | | | 1 |
| 9 | LLFGYPVYV | HTLV tax 11 | 0.36 | | | | | 1 |
| 9 | LLNGYPVYV | HTLV tax F13>N analog | 0.18 | | | | | 1 |
| 8 | ALGTTCYA | HuK2.147 | 0.048 | 0.054 | 0.27 | 0.0008 | 0.012 | 3 |
| 8 | ALPEKPAV | HuK2.235 | 0.0009 | 0.02 | 0.051 | 0.0001 | -0.0001 | 2 |
| 9 | ALSVGCTGA | HuK2.9 | 0.041 | 0.0038 | 0.11 | 0.0066 | -0.0001 | 2 |
| 10 | ALSVGCTGAV | HuK2.9 | 0.018 | 0.26 | 0.4 | 0.0051 | 0.0012 | 3 |
| 10 | AVPLIQSRIV | HuK2.17 | 0.0014 | 0.051 | 0.049 | 0.0035 | 0.0058 | 2 |
| 10 | CARAYSEKVT | HuK2.184 | 0.0074 | 0.071 | 0.02 | 0.003 | 0.0071 | 2 |
| 11 | DLMLLRLSEPV | HuK2.120.V11 | 0.1 | 0.075 | 0.35 | 0.025 | 0.0029 | 4 |
| 10 | DLVLSIALSV | HuK2.3 | 0.001 | 0.018 | 0.0052 | 0.023 | 0.0051 | 2 |
| 10 | DVVKVLGLPT | HuK2.134 | 0.0012 | 0.023 | 0.046 | 0.0004 | 0.0017 | 2 |
| 10 | FLRPRSLQCV | HuK2.165 | 0.041 | 0.094 | 1.1 | 0.0068 | 0.0036 | 3 |
| 9 | FMLCAGLWT | HuK2.195 | 0.022 | 0.0019 | 0.016 | 0.017 | 0.0006 | 2 |
| 10 | GAVPLIQSRI | HuK2.16 | 0.0017 | 0.052 | 0.038 | 0.0041 | 0.0057 | 2 |
| 11 | GLPTQEPALGT | HuK2.140 | 0.0003 | 0.02 | 0.045 | 0.0006 | 0.002 | 2 |
| 10 | GVLVHPQWVL | HuK2.52 | 0.0057 | 0.03 | 0.064 | 0.0045 | 0.0015 | 2 |
| 11 | HLLSNDMCARA | HuK2.177 | 0.029 | 0.052 | 0.11 | 0.0088 | 0.0004 | 4 |
| 10 | IALSVGCTGA | HuK2.8 | 0.0013 | 0.05 | 0.018 | 0.018 | 0.0005 | 2 |
| 11 | IALSVGCTGAV | HuK2.8 | 0.0009 | 0.0032 | 0.027 | 0.01 | 0.0061 | 2 |
| 9 | KITDWKVL | HuK2.131 | 0.0047 | 0.05 | 0.042 | 0.0021 | 0.0002 | 2 |
| 9 | ICVTEFMLCA | HuK2.191 | 0.0035 | 0.0092 | 0.19 | 0.16 | 0.0004 | 3 |
| 11 | KVTEFMLCAGL | HuK2.191 | 0.001 | 0.028 | 0.028 | 0.016 | 0.0036 | 3 |
| 10 | LLRLSEPAKI | HuK2.123 | 0.003 | 0.029 | 0.92 | 0.001 | 0.0008 | 2 |
| 10 | LLSNDMCARA | HuK2.178 | 0.003 | 0.08 | 0.028 | 0.002 | 0.0042 | 2 |
| 10 | LMLLRLSEPA | HuK2.121 | 0.025 | 0.26 | 0.15 | 0.004 | 0.0016 | 3 |
| 10 | LVCNGVLQGI | HuK2.217 | 0.0068 | 0.096 | 0.11 | 0.03 | 0.016 | 3 |
| 11 | LVIIPQWVLTAA | HuK2.54 | 0.003 | 0.015 | 0.49 | 0.03 | 0.0034 | 3 |
| 9 | MLLRLSEPA | HuK2.122 | 0.053 | 0.018 | 0.013 | 0.0081 | 0.0016 | 3 |
| 10 | NMSLLKHQSL | HuK2.102 | 0.0043 | 0.026 | 0.04 | 0.0058 | 0.002 | 2 |
| 10 | PAKITDWKV | HuK2.129 | 0.0011 | 0.01 | 0.032 | 0.0006 | 0.0002 | 2 |
| 9 | PALGTTCYA | HuK2.146 | 0.0083 | 0.021 | 0.027 | 0.0002 | 0.0035 | 2 |
| 11 | PLVCNGVLQGV | HuK2.216.V11 | 0.22 | 0.017 | 0.36 | 0.03 | 0.0049 | 4 |
| 10 | PTQEPALGTT | HuK2.142 | 0.0084 | 0.022 | 0.052 | 0.0037 | 0.0005 | 2 |
| 10 | PVSHSFPHPL | HuK2.91 | 0.0019 | 0.0099 | 0.068 | 0.0022 | 0.0011 | 2 |
| 10 | QVAVYSHGWA | HuK2.39 | 0.0004 | 0.0097 | 0.02 | 0.0005 | 0.0252 | 2 |
| 11 | SLHLLSNDMCA | HuK2.175 | 0.039 | 1.9 | 0.69 | 0.0005 | 0.0004 | 3 |
| 9 | SLQCVSLHL | HuK2.170 | 0.01 | 0.084 | 0.024 | 0.0006 | 0.0031 | 3 |
| 10 | SLQCVSLHLL | HuK2.170 | 0.0099 | 0.4 | 0.092 | 0.0059 | 0.0008 | 2 |
| 11 | SVGCTGAVPLI | HuK2.11 | 0.01 | 0.001 | 0.0007 | 0.0007 | 0.0005 | 1 |
| 8 | VLSIALSV | HuK2.5 | 0.005 | 0.079 | 0.02 | 0.0024 | 0.0003 | 2 |
| 9 | VLVHPQWVL | HuK2.53 | 0.035 | 0.83 | 0.0039 | 0.0014 | 0.0004 | 2 |
| 11 | VLVHPQWVLTA | HuK2.53 | 0.012 | 2.1 | 0.14 | 0.019 | 0.0008 | 4 |
| 11 | VLVHPQWVLTV | HuK2.53.V11 | 0.68 | 2.9 | 3.8 | 0.34 | 0.0018 | 4 |
| 9 | VLVHPQMW | HuK2.53.V9 | 0.011 | 0.14 | 0.0039 | 0.0013 | 0.0005 | 2 |
| 8 | WIKDTIAA | HuK2.252 | 0.0002 | 0.012 | 0.17 | 0.0002 | -0.0001 | 2 |
| 9 | FVEEQMTWV | KSHV.105.V9 | 0.12 | 0.028 | 0.019 | 0.14 | 0.0013 | 3 |
| 9 | LVYHIYSKV | KSF3V.153.V9 | 0.016 | 0.034 | 0.67 | 0.011 | 0.054 | 4 |
| 9 | ALDKAYVLL | analog of A*0207 eluted | 0.093 | | | | | 1 |
| 9 | ALDKYTVLL | analog of A*0207 eluted | 0.068 | | | | | 1 |
| 10 | FLDSDYFPSI | double analog of 941.01 | 0.019 | | | | | 1 |
| 10 | FLDSDYFPSL | double analog of 941.01 | 0.017 | | | | | 1 |
| 10 | FLDSDYFPSV | analog of 941.01 | 0.51 | | | | | 1 |
| 9 | FLDSYIAPL | consensus 2.1/2.7 | 0.21 | | | | | 1 |
| 9 | ALNKMFCQL | p53.129 | 0.0075 | 0.0052 | 0.03 | 0.008 | 0.0004 | 2 |
| 9 | ALDVYNGLL | PAP.299 | 0.0685 | 0.15 | 0.039 | 0.0012 | -0.0003 | 3 |
| 10 | ILYSAHDTTV | PAP.284.L2 | 0.013 | 4.4 | 0.65 | 0.0025 | 0.0014 | 3 |
| 9 | IVLWQPIPV | PAP.135.V2 | 0.013 | 0.0019 | 0.029 | 0.0002 | 0.01 | 2 |
| 10 | LVFFWLDRSV | PAP.21.V2 | 0.0043 | 0.014 | 0.11 | 0.052 | 0.17 | 3 |
| 9 | TLMSAMTNL | PAP.112 | 0.1166 | 5.5 | 3.4 | 0.013 | 0.027 | 4 |
| 9 | TLMSAMTNV | PAP.112.V9 | 0.0083 | 0.24 | 0.3 | 0.0019 | 0. 028 | 2 |
| 10 | IVSVSSFLFV | Pf.CSP.7.V2 | 0.0053 | 0.0055 | 0.052 | 0.05 | 0.02 | 2 |
| 8 | ALGTTCYV | PSA.143.V8 | 0.068 | 0.67 | 0.85 | 0.014 | 0.0003 | 4 |
| 11 | MLLRLSEPAE V | PSA.118.V11 | 0.017 | 0.013 | 0.087 | 0.0021 | 0.0018 | 3 |
| 9 | MLLRLSEPV | PSA.118.V9 | 0.14 | 0.12 | 0.22 | 0.0058 | 0.019 | 3 |
| 9 | AVFDIESKV | PSM.711.V2 | 0.0091 | 0.071 | 0.32 | 0.04 | 0.039 | 3 |
| 10 | GLPEGDLVYV | PSM.168.L2 | 0.12 | 2.1 | 4.8 | 0.033 | 0.0083 | 4 |
| 10 | GLPSIPVHPV | PSM.288.V10 | 0.091 | 6.5 | 17 | 0.012 | 0.066 | 4 |
| 10 | GVPEGDLVYV | PSM.168.V2 | 0.0039 | 0.016 | 0.013 | 0.019 | 0.011 | 2 |
| 10 | QLMFLERAFV | PSM.667.V10 | 0.0095 | 0.42 | 0.21 | 0.0051 | 3.8 | 2 |
| 9 | VLAGGFFLV | PSM.27.V9 | 0.19 | 12 | 2 | 0.065 | 0.037 | 4 |
| 9 | ALSLAAVLV | T. cruzi.7 | 0.0448 | | | | | 1 |
| 10 | ALSLAAVLVV | T. cruzi.7 | 0.0154 | | | | | 1 |
| 10 | AMALIGDSTV | T. cruzi.972 | 0.0338 | | | | | 1 |
| 10 | FLAGHPLTNL | T. cruzi.161 | 0.3547 | | | | | 1 |
| 9 | FLARLHAAA | T. cruzi.67 | 0.1036 | | | | | 1 |
| 9 | FTLVAPVSI | T. cruzi.534 | 0.0138 | | | | | 1 |
| 9 | FTSAVLLLV | T. cruzi.6 | 0.4933 | | | | | 1 |
| 10 | GLLLLGLWGT | T. cruzi.990 | 0.081 | | | | | 1 |
| 10 | GLMNNAFEWI | T. cruzi.188 | 0.0286 | | | | | 1 |
| 10 | IILNGSLLTL | T. cruzi.581 | 0.0249 | | | | | 1 |
| 9 | KLYCSYEVA | T. cruzi.401 | 0.011 | | | | | 1 |
| 10 | KTWADEYLCV | T. cruzi.480 | 0.0113 | | | | | 1 |
| 9 | KVVSLVILA | T. cruzi.236 | 0.0122 | | | | | 1 |
| 10 | LLLEHGQFDL | T. cruzi.961 | 0.1358 | | | | | 1 |
| 10 | LLLGLWGTAA | T. cruzi.992 | 0.0208 | | | | | 1 |
| 9 | LMNNAFEWI | T. cruzi.189 | 0.0127 | | | | | 1 |
| 10 | LMNNAFEWIV | T. cruzi.189 | 0.0886 | | | | | 1 |
| 9 | MTSCVSEQL | T. cruzi.274 | 0.0144 | | | | | 1 |
| 10 | QMDYSNGLFV | T. cruzi.610 | 0.082 | | | | | 1 |
| 9 | RLLSPTTIV | T. cruzi.120 | 0.0172 | | | | | 1 |
| 10 | RVFGLLLLGL | T. cruzi.987 | 0.014 | | | | | 1 |
| 9 | SMNATLVQA | T. cruzi.47 | 0.0187 | | | | | 1 |
| 9 | SVFRENLFL | T. cruzi.60 | 0.0852 | | | | | 1 |
| 9 | TLLYATVEV | T. cruzi.380 | 0.0734 | | | | | 1 |
| 9 | VMACLVPAA | T. cruzi.16 | 0.4916 | | | | | 1 |
| 9 | VMMSCSSEA | T. cruzi.16 | 0.0908 | | | | | 1 |
| 10 | VVMACLVPAA | T. cruzi.15 | 0.0508 | | | | | 1 |
| 9 | WIIKNSWTA | T. cruzi.300 | 0.0269 | | | | | 1 |
| 9 | WLSDCGEAL | T. cruzi.457 | 0.022 | | | | | 1 |
| 10 | YVNGVVVATV | T. cruzi.66 | 0.0107 | | | | | 1 |
| 9 | RLPEPQDVV | TRP1 | 0.013 | | | | | 1 |

### Example 2

Identification of immunogenic peptides Using the B7-like supermotif identified in references described above, sequences from various pathogens and tumor-related proteins were analyzed for the presence of these motifs. Screening was carried out described in the references. Tables 7 and 8 provides the results of searches of the antigens.

**Table 7**

| Peptide | Sequence | Source | B*0702 | B7 XRN |
|---|---|---|---|---|
| 1347.04 | LPVSPRLQI | CEA.363.I9 | 0.011 | 2 |
| 34.0262 | MPNQAQMRII | Her2/neu.706 | 0.0001 | 0 |
| 1347.08 | MPNQAQMRII | Her2/neu.706 | 0.0014 | 2 |
| 34.0263 | MPYGCLLDHI | Her2/neu.801 | 0.0048 | 0 |
| 1347.1 | MPYGCLLDHI | Her2/neu.801 | 0.0013 | 3 |
| 40.006 | APAPAPSW | p53.84 | 0.0062 | |
| 1347.21 | APAPAPSW | p53.84 | 0.011 | 3 |
| 1347.46 | MPNQAQMRILK | Her2/neu.706 | -0.0001 | 0 |
| 1347.47 | MPYGCLLDHVR | Her2/neu.801 | 0.0001 | 1 |
| 40.0165 | LPQHLFGYSW | CEA.58 | -0.0002 | |
| 48.0003 | FPWQRLLI | CEA.13.F1I8 | 0.01 | 4 |
| 48.0004 | FPWQRLLL | CEA.13.F1 | 0.28 | 4 |
| 48.0007 | FPQHLFGI | CEA.58.F1 | 0.014 | 3 |
| 48.0008 | YPNASLLI | CEA.102 | 0.028 | 4 |
| 48.0014 | FPGVNLSL | CEA.428.F1 | 0.29 | 4 |
| 48.0016 | FPQQHTQI | CEA.632.F1 | 0.025 | 3 |
| 48.0017 | FPNNNGTI | CEA.646.F1 | 0.02 | 3 |
| 48.0018 | FPGLSAGI | CEA.680.F1I8 | 0.34 | 4 |
| 48.0024 | FPDSLPDL | HER2/neu.415.F1 | 0.21 | 2 |
| 48.0026 | FPVAIKVL | HER2/neu.748.F1 | 0.041 | 2 |
| 48.0029 | FPYVSRLL | HER2/neu.779.F1 | 0.09 | 3 |
| 48.003 | FPIKWMAI | Her2/neu.884.FlI8 | 0.25 | 3 |
| 48.0031 | FPIKWMAL | HER2/neu.884.F1 | 9.3 | 4 |
| 48.0032 | FPYDGIPA | Her2/neu.921.F1 | 0.013 | 4 |
| 48.0033 | FPYDGIPI | Her2/neu.921.F1I8 | 0.031 | 5 |
| 48.0034 | FPRFRELI | Her2/neu.966.F1I8 | 0.01 | 3 |
| 48.0035 | FPRFRELV | HER2/neu.966.F1 | 0.02 | 2 |
| 48.0036 | FPGAGGMI | Her2/neu.1036.F1 | 0.03 | 2 |
| 48.0038 | FPGKNGVI | Her2/neu.1174.F1I8 | 0.012 | 3 |
| 48.0039 | FPGKNGVV | HER2/neu.1174.F1 | 0.069 | 3 |
| 48.004 | FPISHLYI | MAGE2.170.F1I8 | 0.026 | 4 |
| 48.0041 | FPKTGLLI | NAGE2.196.F1I8 | 0.041 | 3 |
| 48.0042 | FPQGASSI | MAGE3.64.F1 | 0.24 | 3 |
| 48.0043 | FPLWSQSI | MAGE3.77.F1 | 0.045 | 4 |
| 48.0044 | FPKAGLLI | MAGE3.196.F1I8 | 0.18 | 3 |
| 48.0045 | FPLPSQAI | p53.33.F1 | 0.013 | 4 |
| 48.0046 | FPRMPEAA | p53.63.F1 | 0.02 | 2 |
| 48.0047 | FPRMPEAI | p53.63.F1I8 | 0.044 | 3 |
| 48.0049 | FPAAPTPI | p53.76.F1I8 | 0.043 | 3 |
| 48.005 | FPTPAAPI | p53.79.F1I8 | 0.061 | 3 |
| 48.0051 | FPAAPAPI | p53.B1.F1I8 | 0.26 | 2 |
| 48.0052 | FPAPAPSI | p53.84.FH8 | 0.038 | 5 |
| 48.0053 | FPAPSWPI | p53.86.F1I8 | 0.046 | 5 |
| 48.0054 | FPAPSWPL | p53.86.F1 | 0.53 | 5 |
| 48.0056 | FPALNKMI | p53.127.F1I8 | 0.017 | 3 |
| 48.0057 | FPSAPPHRI | CEA.3.F1I9 | 0.11 | 4 |
| 48.0058 | FPHRWCIPI | CEA.7.F1I9 | 2.2 | 5 |
| 48.006 | FPAYSGREI | CEA.92.F1 | 2.6 | 3 |
| 48.0064 | FPVSPRLQI | CEA.363.F1I9 | 0.41 | 4 |
| 48.0065 | FPVSPRLQL | CEA.363.F1 | 8.3 | 4 |
| 48.0066 | FPPAQYSWL | CEA.442.F1 | 0.042 | 2 |
| 48.0067 | FPQQHTQVI | CEA.632.F1I9 | 1.6 | 5 |
| 48.0068 | FPQQHTQVL | CEA.632.F1 | 6.3 | 5 |
| 48.0069 | FPTNASLSF | Her2/neu.65.F1 | 5.1 | 4 |
| 48.007 | FPTNASLSI | HER2/neu.65.F1I9 | 1.2 | 5 |
| 48.0071 | FPLNNTTPI | Her2/neu.121.F1I9 | 3.4 | 5 |
| 48.0072 | FPLNNTTPV | Her2/neu.121.F1 | 30 | 4 |
| 48.0073 | FPSGVKPDI | Her2/neu.600.F1I9 | 0.88 | 3 |
| 48.0074 | FPSGVKPDL | Her2/neu.600.F1 | 9.1 | 2 |
| 48.0075 | FPDLSYMPI | Her2/neu.605.F1 | 0.25 | 5 |
| 48.0076 | FPLTSIISA | Her2/neu.649.F1 | 0.019 | 3 |
| 48.0077 | FPLTSIISI | HER2/neu.649.F1I9 | 0.026 | 5 |
| 48.0078 | EPLTPSGAI | Her2/neu.698.I9 | 0.017 | 2 |
| 48.0079 | FPLTPSGAI | Her2/neu.698.F1I9 | 5.8 | 3 |
| 48.008 | FPLTPSGAM | Her2/neu.698.F1 | 2.8 | 4 |
| 48.0081 | FPNQAQMRI | Her2/neu.706.F1 | 0.081 | 5 |
| 48.0082 | FPKANKEII | Her2/neu.760.F1I9 | 0.65 | 3 |
| 48.0083 | FPKANKEIL | Her2/neu.760.F1 | 0.82 | 3 |
| 48.0084 | FPQPPICTI | Her2/neu.941.F1 | 0.28 | 5 |
| 48.0085 | FPLAPSEGA | Her2/neu.1073.F1 | 0.015 | 2 |
| 48.0086 | FPLAPSEGI | Her2/neu.1073.F1I9 | 0.055 | 4 |
| 48.0087 | FPTHDPSPI | Her2/neu.1101.F1I9 | 0.051 | 4 |
| 48.0088 | FPTHDPSPL | Her2/neu.1101.F1 | 0.55 | 3 |
| 48.0089 | FPSETDGYI | Her2/neu.1120.F1I9 | 0.025 | 4 |
| 48.009 | FPSETDGYV | Her2/neu.1120.F1 | 0.027 | 3 |
| 48.0091 | FPREGPLPA | Her2/neu.1151.F1 | 19 | 2 |
| 48.0092 | FPREGPLPI | HER2/neu.1151.F1I9 | 17 | 3 |
| 48.0093 | FPLPAARPA | Her2/neu.1155.F1 | 0.16 | 3 |
| 48.0094 | FPLPAARPI | HER2/neu.1155.F1I9 | 0.84 | 4 |
| 48.0095 | FPAARPAGA | Her2/neu.1157.F1 | 0.068 | 2 |
| 48.0096 | FPAARPAGI | Her2jneu.1157.F1I9 | 0.59 | 3 |
| 48.0099 | FPQPHPPPA | Her2/neu.1204.F1 | 0.053 | 3 |
| 48.01 | FPQPHPPPI | Her2/neu.1204.F1I9 | 0.073 | 4 |
| 48.0101 | FPPPAFSPA | Her2jneu.1208.F1 | 0.12 | 4 |
| 48.0102 | FPPPAFSPI | Her2/neu.1208.F1I9 | 0.41 | 3 |
| 48.0107 | FPISHLYII | MAGE2.170.F1I9 | 1.9 | 5 |
| 48.0108 | FPISHLYIL | MAGE2.170.F1 | 2.5 | 5 |
| 48.0109 | FPKTGLLII | MAGE2.196.F1 | 0.015 | 4 |
| 48.0112 | FPHISYPPL | MAGE2.2 96. F1 | 3.3 | 5 |
| 48.0113 | FPTTMNYPI | MAGE3.71.F1I9 | 0.094 | 5 |
| 48.0114 | FPTTMNYPL | MAGE3.71.F1 | 0.86 | 5 |
| 48.0115 | LPTTMNYPI | MAGE3.71.I9 | 0.055 | 5 |
| 48.0117 | FPIGHLYII | MAGE3.170.F1I9 | 1.6 | 5 |
| 48.0118 | FPHISYPPI | MAGE3.296.F1I9 | 1.9 | 5 |
| 48.012 | FPPVAPAPI | p53.70.F1I9 | 0.02 | 3 |
| 48.0121 | FPAAPTPAA | p53.76.F1 | 1.3 | 3 |
| 48.0122 | FPAAPTPAI | p53.76.F1I9 | 2.1 | 5 |
| 48.0125 | FPILTIITI | p53.249.F1I9 | 0.074 | 5 |
| 48.0126 | FPILTIITL | p53.249.F1 | 0.85 | 5 |
| 48.0129 | FPPHRWCIPI | CEA.6.F1I10 | 0.056 | 3 |
| 48.0132 | FPWQRLLLTI | CEA.13.F1I10 | 0.019 | 4 |
| 48.0133 | NPPTTAKLTI | CEA.29 | 0.019 | 2 |
| 48.0134 | FPQHLFGYSI | CEA.58.F1I10 | 0.024 | 4 |
| 48.0135 | FPDAPTISPI | CEA.236.F1I10 | 0.03 | 3 |
| 48.0136 | FPDAPTISPL | CEA.236.F1 | 0.15 | 3 |
| 48.0138 | FPQQHTQVLF | CEA.632.F1 | 0.12 | 3 |
| 48.0139 | FPQQHTQVLI | CEA.632.F1I10 | 0.041 | 4 |
| 48.014 | FPGLSAGATI | CEA.680.F1 | 0.026 | 3 |
| 48.0141 | FPTNASLSFI | Her2/neu.65.F1I10 | 0.046 | 5 |
| 48.0142 | FPTNASLSFL | Her2/neu.65.F1 | 0.31 | 5 |
| 48.0143 | FPGGLRELQI | Her2/neu.133.F1I10 | 0.018 | 3 |
| 48.0145 | FPMCKGSRCI | Her2/neu.196.F1 | 0.03 | 3 |
| 48.0146 | FPNPEGRYTI | Her2/neu.282.F1 | 0.017 | 5 |
| 48.0149 | FPCARVCYGL | Her2/neu.336.F1 | 0.029 | 3 |
| 48.015 | FPHQALLHTA | Her2/neu.488.F1 | 0.014 | 3 |
| 48.0151 | FPHQALLHTI | Her2/neu.488.F1I10 | 0.064 | 4 |
| 48.0153 | FPAEQRASPI | Her2/neu.642.F1I10 | 1.8 | 3 |
| 48.0154 | FPAEQRASPL | Her2/neu.642.F1 | 3.8 | 3 |
| 48.0155 | FPLTSIISAI | Her2/neu.649.F1I10 | 0.024 | 5 |
| 48.0156 | FPLTSIISAV | Her2/neu.649.F1 | 0.025 | 4 |
| 48.0157 | FPSGAMPNQI | Her2/neu.701.F1 | 0.024 | 3 |
| 48.0158 | FPNQAQMRII | Her2/neu.706.F1I10 | 0.019 | 3 |
| 48.0159 | FPNQAQMRIL | Her2/neu.706.F1 | 0.068 | 5 |
| 48.016 | FPYVSRLLGI | Her2/neu.779.F1 | 0.049 | 4 |
| 48.0162 | FPYGCLLDHV | Her2/neu.801.F1 | 0.01 | 3 |
| 48.0163 | FPASPLDSTF | Her2/neu.995.F1 | 1.5 | 4 |
| 48.0164 | FPASPLDSTI | Her2/neu.995.F1I10 | 0.28 | 5 |
| 48.0165 | FPREGPLPAA | Her2/neu.1151.F1 | 0.13 | 2 |
| 48.0166 | FPREGPLPAI | HER2/neu.1151.F1I10 | 0.27 | 3 |
| 48.0167 | FPQPHPPPAF | Her2/neu.1204.F1 | 0.026 | 5 |
| 48.0168 | FPQPHPPPAI | Her2/neu.1204.F1I10 | 0.011 | 5 |
| 48.0169 | FPPPAFSPAF | Her2/neu.1208.F1 | 0.02 | 4 |
| 48.0172 | FPISHLYILI | MAGE2.170.F1I10 | 0.057 | 5 |
| 48.0173 | FPISHLYILV | MAGE2.170.F1 | 0.053 | 5 |
| 48.0176 | FPRKLLMQDI | MAGE2.241.F1I10 | 1.1 | 2 |
| 48.0177 | FPRKLLMQDL | MAGE2.241.F1 | 0.91 | 2 |
| 48.0179 | FPTTMNYPLW | MAGE3. 71.F1 | 0.025 | 4 |
| 48.018 | FPIGHLYIFA | MAGE3.170.F1 | 0.14 | 5 |
| 48.0181 | FPIGHLYIFI | MAGE3.170.F1I10 | 0.087 | 5 |
| 48.0182 | FPKAGLLIII | MAGE3.196.F1I10 | 0.027 | 3 |
| 48.0183 | FPKAGLLIIV | MAGE3.196.F1 | 0.025 | 3 |
| 48.0184 | FPPVAPAPAA | p53.70.F1 | 0.011 | 2 |
| 48.0185 | FPPVAPAPAI | p53.70.F1I10 | 0.019 | 3 |
| 48.0186 | FPAPAAPTPA | p53.74.F1 | 7.2 | 3 |
| 48.0187 | FPAPAAPTPI | p53.74.F1I10 | 3.7 | 5 |
| 48.0188 | FPTPAAPAPI | p53.79.F1 | 0.011 | 3 |
| 48.0189 | FPAPAPSWPI | p53.84.F1I10 | 0.44 | 5 |
| 48.019 | FPAPAPSWPL | p53.84.F1 | 1.3 | 4 |
| 48.0191 | FPSWPLSSSI | p53.88.F1I10 | 1 | 5 |
| 48.0192 | FPSWPLSSSV | p53.88.F1 | 1.7 | 5 |
| 48.0195 | FPPGSTKRAI | p53.299.F1I10 | 0.026 | 2 |
| 48.0201 | FPSGVKPDLSI | Her2/neu.600.F1I11 | 0.028 | 4 |
| 48.0203 | FPLTSIISAVI | Her2/neu.649.F1I11 | 0.015 | 5 |
| 48.0206 | FPYDGIPAREI | HER2/neu.921.F1I11 | 0.039 | 4 |
| 48.0207 | FPRFRELVSEF | Her2/neu.966.F1 | 0.071 | 2 |
| 48.0208 | FPRFRELVSEI | Her2/neu.966.F1I11 | 0.024 | 5 |
| 48.0209 | FPLDSTFYRSI | Her2/neu.998.F1I11 | 0.024 | 3 |
| 48.021 | FPLDSTFYRSL | HER2/neu.998.F1 | 0.13 | 4 |
| 48.0211 | FPSPREGPLPA | Her2/neu.1149.F1 | 0.078 | 2 |
| 48.0212 | FPSPREGPLPI | Her2/neu.1149.F1I11 | 0.029 | 3 |
| 48.0213 | FPLPAARPAGA | Her2/neu.1155.F1 | 0.025 | 2 |
| 48.0215 | FPAARPAGATI | Her2/neu.1157.F1I11 | 0.14 | 4 |
| 48.0216 | FPAARPAGATL | HER2/neu.1157.F1 | 1.4 | 4 |
| 48.0219 | FPRKLLMQDLI | MAGE2.241.F1 | 0.064 | 3 |
| 48.022 | FPRALIETSYI | MAGE2.274.F1I11 | 0.74 | 4 |
| 48.0221 | FPRALIETSYV | MAGE2.274.F1 | 0.87 | 3 |
| 48.0223 | FPKAGLLIIVL | MAGE3.196.F1 | 0.013 | 2 |
| 48.0224 | FPRALVETSYI | MAGE3.274.F1I11 | 0.76 | 3 |
| 48.0225 | FPRALVETSYV | MAGE3.274.F1 | 1.3 | 3 |
| 48.0226 | FPRMPEAAPPI | p53.63.F1I11 | 0.14 | 3 |
| 48.0227 | FPRMPEAAPPV | p53.63.F1 | 0.23 | 3 |
| 48.0228 | FPAPAAPTPAA | p53.74.F1 | 0.063 | 3 |
| 48.023 | FPAAPTPAAPA | p53.76.F1 | 0.16 | 3 |
| 48.0231 | FPAAPTPAAPI | p53.76.F1I11 | 0.11 | 3 |
| 48.0232 | FPSQKTYQGSI | p53.97.F1 | 0.011 | 2 |
| 48.0233 | FPALNKMFCQI | p53.127.F1I11 | 0.017 | 2 |
| 1347.02 | LPQHLFGYSW | CEA.58 | -0.0001 | 1 |

**Table 8**

| AA | Sequence | Source | B*0702 | B*3501 | B*5101 | B*5301 | B*5401 | B7 XRN |
|---|---|---|---|---|---|---|---|---|
| 9 | APIEHIASM | Flu.RRP2.433 | 0.52 | | | | | 1 |
| 9 | APIMFSNKM | Flu.RRP1.340 | 0.46 | | | | | 1 |
| 10 | APPKQSRMQF | Flu.RRP3.624 | 0.23 | | | | | 1 |
| 9 | APSPYNSRF | Flu.NRAM.151 | 0.29 | | | | | 1 |
| 10 | CPKYVRSAKL | Flu.HEMA.320 | 0.016 | | | | | 1 |
| 10 | FPNEVGARIL | Flu.RRP3.168 | 0.015 | | | | | 1 |
| 10 | FPSSSYRRPV | Flu.RRP1.700 | 0.26 | | | | | 1 |
| 9 | GPALSINEL | Flu.RRP3.705 | 0.046 | | | | | 1 |
| 9 | GPATAQMAL | Flu.RRP1.540 | 0.16 | | | | | 1 |
| 10 | GPDNGAVAVL | Flu.NRAM.182 | 0.011 | | | | | 1 |
| 10 | GPPCSQRSKF | Flu.RRP2.273 | 0.02 | | | | | 1 |
| 9 | GPVHFRNQV | Flu.RRP3.131 | 0.077 | | | | | 1 |
| 9 | IPAEMLASI | Flu.RRP1.368 | 0.068 | | | | | 1 |
| 10 | IPKQKVAGPL | Flu.NS1.106 | 0.13 | | | | | 1 |
| 9 | IPSIQSRGL | Flu.HEMA.338 | 0.018 | | | | | 1 |
| 10 | LPACVYGPAV | Flu.VNUC.276 | 0.025 | | | | | 1 |
| 9 | LPFDRTTVM | Flu.VNUC.418 | 2.3 | | | | | 1 |
| 10 | LPRRSGAAGA | Flu.VNUC.172 | 0.072 | | | | | 1 |
| 9 | LPSLPGHTA | Flu.NS1.163 | 0.13 | | | | | 1 |
| 10 | LPVRSWSYIV | Flu.HEMA.88 | 0.027 | | | | | 1 |
| 10 | MPAHGPAKNM | Flu.RRP1.646 | 0.045 | | | | | 1 |
| 9 | NPALRMKWM | Flu.RRP3.42 | 0.092 | | | | | 1 |
| 9 | NPRMFLAMI | Flu.RRP1.314 | 0.54 | | | | | 1 |
| 9 | QPEWFRNVL | Flu.RRP1.329 | 0.22 | | | | | 1 |
| 9 | RPCFWVELI | Flu.NRAM.404 | 0.082 | | | | | 1 |
| 9 | RPLLIEGTA | Flu.RRP1.393 | 0.018 | | | | | 1 |
| 9 | RPVGISSMV | Flu.RRP1.707 | 0.12 | | | | | 1 |
| 10 | RPWVSFDQNL | Flu.NRAM.286 | 0.014 | | | | | 1 |
| 9 | SPGMMMGMF | Flu.RRP1.404 | 0.04 | | | | | 1 |
| 9 | SPIVPSFDM | Flu.VNUC.473 | 0.11 | | | | | 1 |
| 10 | SPKGVEESSI | Flu.RRP2.624 | 0.026 | | | | | 1 |
| 9 | SPLMVAYML | Flu.RRP3.199 | 0.12 | | | | | 1 |
| 10 | SPYNSRFESV | Flu.NRAM.153 | 0.13 | | | | | 1 |
| 10 | TPLGAINSSL | Flu.HEMA.298 | 0.16 | | | | | 1 |
| 10 | VPASRYLTDM | Flu.NS1.84 | 0.4 | | | | | 1 |
| 9 | YPKIYKTYF | Flu.RRP3.111 | 0.15 | | | | | 1 |
| 9 | YPKLKNSYV | Flu.HEMA.175 | 0.031 | | | | | 1 |
| 10 | FPHCLAFSYI | HBV pol 541 analog | 0.0063 | 0.021 | 0.28 | 0.14 | 0.13 | 4 |
| 8 | HPAAMPHI | B7 HBV fix 20 | 0.0002 | -0.0003 | 0.29 | 0.0009 | 0.25 | 2 |
| 9 | HPAAMPHLI | B7 HBV fix 3 | 0.0032 | 0.0005 | 0.034 | 0.028 | 0.61 | 3 |
| 10 | IPIPSSWAFI | HBV env 324 analog | 0.0028 | 0.0097 | 0.59 | 0.078 | 0.018 | 2 |
| 8 | LPIFFCLI | HBV.env.379 | 0.0004 | 0.0004 | 0.45 | 0.029 | 0.06 | 3 |
| 9 | LPIFFCLWI | HBV.env.379 | -0.0001 | 0.0031 | 0.62 | 0.39 | 0.22 | 3 |
| 9 | LPIHTAELI | HBV.pol.712 | 0.0005 | 0.0047 | 0.13 | 0.75 | 0.0074 | 2 |
| 11 | LPIHTAELLAI | HBV.pol.712 | 0.0015 | 0.0006 | 0.11 | 0.023 | 0.033 | 3 |
| 9 | LPSDFFPSI | HBV core 19 analog | 0.0008 | 0.019 | 2.3 | 1.7 | 0.3 | 4 |
| 9 | LPVCAFSSI | B7 HBV fix 6 | 0.017 | 0.02 | 0.39 | 0.11 | 0.1.6 | 5 |
| 8 | SPFLLAQI | HBV.pol.511 | 0.17 | -0.0003 | 0.48 | -0.0001 | 0.33 | 3 |
| 10 | TPARVTGGVI | B7 HBV fix 17 | 0.02 | -0.0003 | 0.033 | -0.0001 | 0.0099 | 2 |
| 10 | VPFVQWFVGI | HBV.env.340 | -0.0001 | -0.0003 | 0.03 | -0.0001 | 0.05 | 2 |
| 8 | YPALMPLI | HBV.po1.640 | 0.044 | 0.003 | 0.73 | 0.089 | 8.5 | 4 |
| 9 | YPALMPLYI | B7 HBV fix 9 | 0.018 | 0.011 | 1.1 | 1.4 | 0.2 | 4 |
| 8 | APTLWARI | HCV..2869 | 0.013 | -0.0003 | 0.0054 | -0.0001 | 0.0007 | 1 |
| 9 | FPYLVAYQI | HCV.NS3.1588 | 0.0014 | 0.072 | 0.73 | 1.7 | 9.5 | 4 |
| 8 | IPLVGAPI | HCV..137 | 0.015 | -0.0003 | 3.8 | 0.0013 | 0.093 | 3 |
| 10 | IPQAVVDMVI | HCV.El.340 | 0.0008 | 0.0054 | 0.1 | 0.071 | 0.036 | 3 |
| 10 | LPAILSPGAI | HCV.NS4.1888 | 0.0005 | -0.0003 | 0.021 | -0.0001 | 0.067 | 2 |
| 10 | LPGCSFSIFI | HCV.core.168 | 0.0005 | 0.0004 | 0.038 | 0.032 | 0.011 | 2 |
| 8 | LPRRGPRI | HCV..37 | 0.11 | -0.0003 | 0.021 | -0.0001 | 0.0025 | 2 |
| 10 | YPCTVNFTII | HCV.NS1/E2.623 | 0.0008 | 0.004 | 0.098 | 0.12 | 0.23 | 3 |
| 8 | FPGGLREI | HER2/neu.133. | 0.025 | -0.0002 | 0.0016 | -0.0001 | 0.0049 | 1 |
| | | F1I8 | | | | | | |
| 8 | FPGGLREL | HER2/neu.133. F1 | 0.17 | 0.0005 | -0.0002 | -0.0001 | 0.001 | 1 |
| 10 | FPGGLRELQL | Her2/neu.133. F1 | 0.042 | -0.0002 | -0.0001 | 0.0035 | 0.0064 | 1 |
| 8 | FPKANKEI | HER2/neu.760. F1I8 | 0.16 | -0.0002 | 0.0022 | -0.0001 | 0.0032 | 1 |
| 8 | LPSETDGI | Her2/neu.1120 .I8 | 0.01 | -0.0001 | 0.011 | -0.0002 | 0.0003 | 2 |
| 9 | FPISPIETI | HIV POL 179 | 0.0041 | 0.2 | 0.28 | 0.54 | 6.3 | 4 |
| 9 | FFVRPQVPG | HIV.nef.84.G9 | 0.0058 | 0.095 | 0.0001 | 0.0014 | 5.2 | 2 |
| 9 | FPVRPQVPT | HIV.nef.84.T9 | 0.0067 | 0.024 | 0.0003 | 0.0002 | 5.4 | 2 |
| 9 | FPVRPQVPY | HIV.nef.84.Y9 | 0.0033 | 0.11 | 0.0001 | 0.052 | 0.0059 | 2 |
| 9 | IPIHYCAPI | HIV env 293 analog | 0.016 | 0.0044 | 0.15 | 0.038 | 0.063 | 4 |
| 8 | VPLQLPPI | HIV.rev.71 | 0.001 | -0.0003 | 0.6501 | -0.0001 | 3.1 | 2 |
| 9 | YPLASLRSI | HIV.gag.507 | 0.03 | 0.0006 | 0.62 | 0.0092 | 0.2 | 3 |
| 9 | IPYCNYSKY | Lassa.Josiah CP).361 | 0.0001 | 0.042 | -0.0002 | 0.047 | 0.0004 | 2 |
| 11 | MPKTGLLIIVL | MAGE2 196 | 0.0099 | 0.0018 | 0.0048 | 0.051 | 0.046 | 2 |
| 9 | FPKAGLLII | MAGE3.196.F1 | 0.0094 | 0.0055 | 1.0233 | 0.155 | 0.0665 | 3 |
| 9 | MPKAMLLII | MAGE3.196.M5 | 0.0036 | 0.0002 | 1.02 | 0.05 | 0.424 | 3 |
| 8 | FPALNKMF | p53.127.F1 | 0.025 | 0.0024 | 0.0003 | 0.0015 | 0.0026 | 1 |
| 11 | FPALNKMFCQL | p53.127.F1 | 0.052 | 0.0029 | 0.001 | 0.0012 | 0.02 | 2 |
| 9 | FPGTRVRAI | p53.152.F1 | 1.1 | -0.0002 | 0.0083 | 0.0004 | 0.0041 | 1 |
| 10 | FPPGSTKRAL | p53.299.F1 | 0.79 | -0.0003 | 0.0009 | -0.0004 | -0.0005 | 1 |
| 9 | FPQPKKKPI | p53.315.F1I9 | 0.61 | -0.0002 | -0.0001 | -0.0003 | 0.0006 | 1 |
| 9 | FFQPKKKPL | p53.315.F1 | 2.3 | -0.0002 | -0.0001 | -0.0003 | -0.0001 | 1 |
| 10 | TPYAGEPAPI | Pf SSP2 539 analog | 0.024 | 0.0051 | 0.48 | 0.057 | 0.065 | 4 |

### Example 3

### Identification of immunogenic peptides

Using the A1 motif identified in the references described above, sequences from various pathogens and tumor-related proteins were analyzed for the presence of these motifs. Screening was carried out described in the references.
Table 9 provides the results of searches of the antigens.

**Table 9**

| Peptide | AA | Sequence | Source | A*0101 |
|---|---|---|---|---|
| 1373.78 | 8 | ASFCGSPY | HBV.pol.166 | 0.1000 |
| 1373.88 | 11 | LTFGRETVLEY | HBV.core.137 | 0.3100 |
| 1388.01 | 8 | TTGRTSLY | HBV.pol.798 | 0.0790 |
| 1388.02 | 8 | YSLNFMGY | HPV.pol.580 | 0.0660 |
| 1370.19 | 11 | LADGGCSGGAY | HCV.Entire.1305 | 0.4100 |
| 1370.15 | 11 | LTCGFADLMGY | HCV.Entire.126 | 2.3000 |
| 1370.04 | 9 | LTDPSHITA | HCV.Entire.2180 | 5.1000 |
| 1370.13 | 10 | LVDILAGYGA | HCV.Entire.1853 | 0.0600 |
| 1370.20 | 11 | PTLHGPTPLLY | HCV.Entire.1621 | 0.0550 |
| 1370.24 | 10 | VAATLGFGAY | HCV.NS3.1263 | 0.1300 |
| 1370.05 | 10 | VIDTLTCGFA | HCV.Entire.122 | 0.1400 |
| 1404.08 | 10 | EPSDKHIEQY | Pf.CSP.345 | 0.0750 |
| 1404.02 | 9 | LLACAGLAY | Pf.SSP2.510 | 5.1000 |
| 1404.22 | 8 | LLSTNLPY | Pf.SSP2.121 | 0.0940 |
| 1381.04 | 9 | PSDKFIIEQY | Pf.CSP | 1.7000 |
| 1381.01 | 9 | PSDKHIKEY | Pf.CSP.(MD7) | 1.1000 |
| 1404.05 | 9 | QDEENIGIY | Pf.LSA.1795 | 9.3000 |

### Example 4

Using the A3 supermotif described above, sequences from various pathogens and tumor-related proteins were analyzed for the presence of these motifs. Screening was carried out. Table 10 provides the results of searches of the antigens.

**Table 10**

| AA | Sequence | Source | A*0301 | A*1101 | A*3101 | A*3301 | A*6801 | A3 XRN |
|---|---|---|---|---|---|---|---|---|
| 9 | HVQVLFIAR | CEA.636.V2R9 | 0.0011 | 0.0005 | 0.021 | 0.096 | 0.02 | 2 |
| 11 | DLLDTASALYR | HBV.core.205 | 0.0042 | -0.0003 | -0.0012 | 3.7 | 0.041 | 2 |
| 11 | ELLAACFARSR | HBV.pol.729 | 0.0062 | 0.0016 | 0.02 | 0.2 | 0.16 | 2 |
| 11 | TAELLAACFAR | HEV.pol.727 | 0.0006 | 0.0023 | 0.0066 | 0.16 | 0.059 | 2 |
| 11 | TLWKAGILYKR | HBV.pol.150 | 0.0082 | 0.0095 | 0.1 | 0.11 | 0.064 | 3 |
| 9 | MLQKEYMER | Lassa.gp.414 | -0.001 | 0.0044 | 0.18 | 0.15 | 0.24 | 3 |

### Example 5

Identification of immunogenic peptides Using the A24 motif described above, sequences from various pathogens and tumor-related proteins were analyzed for the presence of these motifs. Screening was carried out described in the references. Table 11 provides the results of searches of the antigens.

**Table 11**

| Peptide | AA | Sequence | Source | A*2402 |
|---|---|---|---|---|
| 1370.64 | 10 | FWAKHMWNFI | HCV NS4 1765 | 0.14 |
| 1370.51 | 11 | LWARMILMTHF | HCV Entire 2872 | 0.052 |
| 1370.56 | 9 | QWMNRLIAF | HCV NS4 1919 | 0.068 |
| 1370.58 | 9 | RMILMTHFF | HCV NS5 2875 | 1.1 |
| 1370.32 | 8 | TYSTYGKF | HCV Entire 1297 | 0.062 |
| 1370.40 | 8 | VMGSSYGF | HCV Entire 2639 | 0.36 |
| 1370.61 | 10 | YYRGLDVSVI | HCV NS3 1422 | 0.027 |

## Claims

1. A composition comprising at least one peptide, the peptide comprising an epitope consisting of the sequence FQPSDYFPSV.

2. A composition of claim 1, wherein the epitope is joined to an amino acid linker.

3. A composition of claim 1, wherein the epitope is admixed with or joined to a CTL epitope.

4. A composition of claim 1, wherein the epitope is admixed with or joined to an HTL epitope.

5. A composition of claim 4, wherein the HTL epitope is a pan-DR binding molecule.

6. A composition of claim 1, further comprising a liposome, wherein the epitope is on or within the liposome.

7. A composition of claim 1, wherein the epitope is joined to a lipid.

8. A composition of claim 1, wherein the peptide is a heteropolymer.

9. A composition of claim 1, wherein the peptide is a homopolymer.

10. A composition of claim 1, wherein the epitope is bound to an HLA heavy chain, β2-microglobulin, and streptavidin complex, whereby a tetramer is formed.

11. A composition of claim 1, further comprising an antigen presenting cell, wherein the epitope is on or within the antigen presenting cell.

12. A composition of claim 11, wherein the epitope is bound to an HLA molecule on the antigen presenting cell, whereby when an A2-restricted cytotoxic lymphocyte (CTL) is present, a receptor of the CTL binds to a complex of the HLA molecule and the epitope.

13. A composition of claim 11, wherein the antigen presenting cell is a dendritic cell.

14. A composition according to any one of the prececing claims for use as a pharmaceutical.

15. A composition according to any one of claims 1-13, for use in a method of inducing a cytotoxic T cell response against a preselected antigen in a patient expressing a specific MHC class I allele, the method comprising contacting cytotoxic T cells from the patient with the composition of claim 1.

## Patentansprüche

1. Zusammensetzung, welche mindestens ein Peptid umfasst, wobei das Peptid ein Epitop umfasst, bestehend aus der Sequenz FQPSDYFPSV.

2. Zusammensetzung nach Anspruch 1, wobei das Epitop mit einem Aminosäurelinker verbunden ist.

3. Zusammensetzung nach Anspruch 1, wobei das Epitop einem CTL-Epitop beigemischt oder mit einem CTL-Epitop verbunden ist.

4. Zusammensetzung nach Anspruch 1, wobei das Epitop einem HTL-Epitop beigemischt oder mit einem HTL-Epitop verbunden ist.

5. Zusammensetzung nach Anspruch 4, wobei das HTL-Epitop ein Pan-DR-bindendes Molekül ist.

6. Zusammensetzung nach Anspruch 1, welches ferner ein Liposom umfasst, wobei das Epitop auf oder innerhalb des Liposoms ist.

7. Zusammensetzung nach Anspruch 1, wobei das Epitop mit einem Lipid verbunden ist.

8. Zusammensetzung nach Anspruch 1, wobei das Peptid ein Heteropolymer ist.

9. Zusammensetzung nach Anspruch 1, wobei das Peptid ein Homopolymer ist.

10. Zusammensetzung nach Anspruch 1, wobei das Epitop an eine schwere HLA-Kette, β2-Mikroglobulin, und einen Streptavidin-Komplex gebunden ist, wobei ein Tetramer gebildet wird.

11. Zusammensetzung nach Anspruch 1, ferner umfassend eine antigenpräsentierende Zelle, wobei das Epitop auf oder innerhalb der antigenpräsentierenden Zelle ist.

12. Zusammensetzung nach Anspruch 11, wobei das Epitop an ein HLA-Molekül auf der antigenpräsentierenden Zelle gebunden ist, wodurch wenn ein A2-restringierter zytotoxischer Lymphozyt (HTL) vorhanden ist, ein Rezeptor des CTL an einen Komplex aus dem HLA-Molekül und dem Epitop bindet.

13. Zusammensetzung nach Anspruch 11, wobei die antigenpräsentierende Zelle eine dendritische Zelle ist.

14. Zusammensetzung nach einem der vorangehenden Ansprüche zur Verwendung als Arzneimittel.

15. Zusammensetzung nach einem der Ansprüche 1 - 13, zur Verwendung in einem Verfahren zur Induktion einer zytotoxischen T-Zellantwort gegen ein vorausgewähltes Antigen in einem Patienten, der ein spezifisches MHC Klasse-I-Allel exprimiert, wobei das Verfahren das Inkontaktbringen von zytotoxischen T-Zellen des Patienten mit der Zusammensetzung von Anspruch 1 umfasst.

## Revendications

1. Composition comprenant au moins un peptide, ce peptide comprenant un épitope consistant en la séquence FQPSDYFPSU.

2. Composition selon la revendication 1, dans laquelle l'épitope est rattaché à un lieur aminoacide.

3. Composition selon la revendication 1, dans laquelle l'épitope est mélangé ou rattaché à un épitope CTL.

4. Composition selon la revendication 1, dans laquelle l'épitope est mélangé ou rattaché à un épitope HTL.

5. Composition selon la revendication 4, dans laquelle l'épitope HTL est une molécule de liaison pan-DR.

6. Composition selon la revendication 1, comprenant en outre un liposome, dans laquelle l'épitope est sur ou à l'intérieur de liposome.

7. Composition selon la revendication 1, dans laquelle l'épitope est rattaché à un lipide.

8. Composition selon la revendication 1, dans laquelle le peptide est un hétéropolymère.

9. Composition selon la revendication 1, dans laquelle le peptide est un homopolymère.

10. Composition selon la revendication 1, dans laquelle l'épitope est lié à une chaîne lourde HLA, une β2-microglobuline et un complexe streptovidine, pout former un tétramère.

11. Composition selon la revendication 1, comprenant en outre une cellule présentant un antigène dans laquelle l'épitope est sur ou à l'intérieur de la cellule présentant l'antigène.

12. Composition selon la revendication 11, dans laquelle l'épitope est rattaché à une molécule HLA sur la cellule présentant l'antigène, et où quand un lymphocyte cytotoxique A2-restreint (CTL) est présent, un récepteur du CTL se lie à un complexe de la molécule HLA et l'épitope.

13. Composition selon la revendication 11, dans laquelle la cellule présentant l'antigène est une cellule dendritique.

14. Composition selon l'une des revendications précédentes, pour utilisation comme produit pharmaceutique.

15. Composition selon l'une des revendications 1 à 13, pour utilisation dans une méthode qui induit une réponse de cellules cytotoxiques T contre un antigène présélectionné dans un patient exprimant un allèle spécifique MHC de classe 1, la méthode comprenant la mise en contact de cellules cytotoxiques T du patient avec la composition de la revendication 1.
